# EUROPÄISCHE PATENTSCHRIFT

(11) **EP 4 311 560 B1**
(45) Veröffentlichungstag und Bekanntmachung des Hinweises auf die Patenterteilung: **25.12.2024**
(21) Anmeldenummer: 22187101.5
(22) Anmeldetag: 26.07.2022
(51) Int. Cl.: A61L 2/10, A61L 2/20, A61L 2/24

(54) **VERFAHREN ZUM EINSCHLEUSEN VON ARTIKELN AUS EINEM GEBINDE IN DIE PROZESSKAMMER EINES CONTAINMENTS UNTER ASEPTISCHEN BEDINGUNGEN UND SCHLEUSENANORDNUNG DAZU**
METHOD FOR FEEDING IN ARTICLES FROM A CONTAINER INTO THE PROCESS CHAMBER OF A CONTAINER UNDER ASEPTIC CONDITIONS AND SLUICE ARRANGEMENT THEREFOR
PROCÉDÉ D'INTRODUCTION DES ARTICLES PROVENANT D'UN RÉCIPIENT DANS LA CHAMBRE DE TRAITEMENT D'UNE ENCEINTE DE CONFINEMENT DANS DES CONDITIONS ASEPTIQUES ET DISPOSITIF D'INTRODUCTION CORRESPONDANT

(43) Veröffentlichungstag der Anmeldung: 31.01.2024
(60) Teilanmeldung: 24192002.4
(73) Patentinhaber: SKAN AG, 4123 Allschwil (CH)
(72) Erfinder: Carli, Julia Annette, 4053 Basel (CH); Leuenberger, Philipp, 4052 Basel (CH); Müller, Mathieu, 68510 Sierentz (FR); Rethoret, Christophe, 68500 Issenheim (FR); Schneidler, Tobias Jan, 6332 Hagendorn (CH); Tondera, Marc, 4125 Riehen (CH); Zeller, Mike, 4246 Wahlen (CH)
(74) Vertreter: Maucher Jenkins Patent- und Rechtsanwälte

(56) Entgegenhaltungen:
- WO-A2-2020/016645
- BAESSLER H J ET AL: "Chapter 6 - Aseptic Transfer Systems Into and Out of Barrier Isolators and RABS", 1 January 2013 (2013-01-01), XP009512843, ISBN: 978-3-642-39291-7, Retrieved from the Internet <URL:https://www.springer.com/gp/book/9783642392917>

## Beschreibung

### Anwendungsgebiet der Erfindung

Die vorliegende Erfindung betrifft ein Verfahren und eine in einem Aufstellraum positionierte Schleusenanordnung zum Einschleusen von Artikeln unter aseptischen Bedingungen aus seriell zugeführten Gebinden in eine von einem Gehäuse umgebene Prozesskammer eines Containments. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist. Die im Innenraum gelagerten Artikel sollen nach dem Öffnen der Abdeckung in der Prozesskammer behandelt werden. Die Behältnisse der Gebinde haben die Gestalt wannenförmiger Tubs, und die Artikel sind z.B. pharmazeutische Phiolen, Vials oder Spritzen, die in systematisch angeordneten muldenförmigen Aufnahmekonturen von Nestern stecken.

### Stand der Technik

Aus der WO 2020/016 645 A2 ist eine Anordnung zum kontaminationsfreien Einschleusen eines sterilen Objektes aus einem Behältnis, welches mit einer semipermeablen Abdeckung verschlossen ist, in eine von einer Wandung umgebenen Arbeitskammer eines Containments bekannt. Die Anordnung umfasst zunächst eine Toreinheit mit einem in der Wandung angeordneten Zugangsflansch, der einen Durchgang von aussen in die Arbeitskammer ausbildet, und einer den Durchgang dicht verschliessenden Tür, die sich zur Offenstellung in die Arbeitskammer bewegen lässt. Zur Anordnung gehört ferner eine Behältnisaufnahme mit einem Auffang zur Halterung eines in die Behältnisaufnahme eingebrachten Behältnisses, einer Öffnung zum Einbringen des Behältnisses in den Auffang und einem Flansch zum Zusammenwirken mit dem Zugangsflansch.

Eine Dekontaminationseinheit ist dazu vorgesehen, bei geschlossener Tür, am Zugangsflansch angedockter Behältnisaufnahme und in der Behältnisaufnahme gelagertem Behältnis, eine der Tür zugewandte Aussenfläche der Abdeckung zu dekontaminieren. In Schliessstellung ist die Tür vonseiten der Arbeitskammer abgedichtet an den Zugangsflansch angefügt, und der Zugangsflansch umrandet einen Zwischenraum, der im Durchgang liegt. Die Abdeckung des in der Behältnisaufnahme eingestellten Behältnisses ist bei an den Zugangsflansch herangeschwenkter Behältnisaufnahme zwischen dem Flansch und dem Zugangsflansch abgedichtet. Die Dekontaminationseinheit ist mit Wirkungsrichtung in den Durchgang hinein direkt an der Tür oder seitlich der Toreinheit installiert, welche die Tür und den Zugangsflansch aufweist. Die Dekontaminationseinheit ist als Strahlungsquelle, z.B. UVC, UV, E-Beam oder Puls-Licht, oder als Begasungseinrichtung, z.B zur Verneblung einer H₂O₂-Lösung ausgebildet.

### Aufgabe der Erfindung

Angesichts der beschränkten Produktivität der gemäss vorbekanntem Stand der Technik existierenden Vorrichtungen und praktizierten Verfahren, liegt der Erfindung die Aufgabe zugrunde, ein für das hiesige Anwendungsgebiet effizienteres Verfahren zum Einschleusen von Artikeln aus einem Gebinde in die Prozesskammer eines Containments unter aseptischen Bedingungen und eine dazu geeignete Schleusenanordnung vorzuschlagen. Massgeblich hierbei sind ein möglichst hoher Automatisierungsgrad, die weitgehende Vermeidung manuellen Eingreifens und Gewährleistung hoher Reinheitserfordernisse. Ausserdem ist darauf zu orientieren, dass bei möglichst geringem zeitlichem und apparativem Dekontaminationsaufwand möglichst nur Artikel in die Prozesskammer transferiert werden, die zu verarbeiten sind.

### Übersicht über die Erfindung

Das erfindungsgemässe Verfahren bzw. die erfindungsgemässe Schleusenanordnung ist zum Einschleusen von Artikeln unter aseptischen Bedingungen aus seriell zugeführten Gebinden in eine von einem Gehäuse umgebene Prozesskammer eines Containments mittels einer in einem Aufstellraum positionierten Schleusenanordnung konzipiert. Der Prozesskammer ist eine Eingangsstation vorgelagert. Ein einzelnes Gebinde umfasst ein Behältnis mit einem aseptischen Innenraum und einem Durchlass, der mit einer Abdeckung verschlossen ist, wobei die im Innenraum gelagerten Artikel nach dem Öffnen der Abdeckung in der Prozesskammer zu behandeln sind.

Im Produktionsmodus der Schleusenanordnung werden die aufeinanderfolgenden Verfahrensschritte ausgeführt:
- zum Transfer der Artikel aus den Gebinden in die Prozesskammer durch von der Eingangsstation sind zumindest zwei in die Prozesskammer führende Passagen vorgesehen und jede der Passagen wird einerseits von einem in der Prozesskammer herangeführten Deckel und/oder andererseits von einem in der Eingangsstation herangeführten Gebinde mit dessen der Passage zugewandten Abdeckung verschlossen;
- bei an die Passage herangeführtem Gebinde wird die der Passage zugewandte Oberfläche der Abdeckung mit eingeschlossenem Umfeld mittels einer Dekontaminationsvorrichtung dekontaminiert;
- nach erfolgter Dekontamination der der Passage zugewandten Oberfläche der Abdeckung, einschliesslich deren Umfeld, wird die Abdeckung geöffnet, um die Artikel aus dem Behältnis durch dessen Durchlass in die Prozesskammer zu transferieren; und
- nach Entleerung des Behältnisses wird zunächst der Deckel wieder an die betreffende Passage herangebracht, um anschliessend das von den Artikeln geleerte Behältnis von der Passage zu entfernen; wobei
- der Deckel an den Passagen im Wechsel zum Einsatz kommt.

Nachfolgend werden spezielle Ausführungsformen des erfindungsgemässen Verfahrens bzw. der Schleusenanordnung definiert:
Die Dekontaminationsvorrichtung ist im Deckel integriert oder ausserhalb des Deckels angeordnet, wobei das Emissionselement der Dekontaminationsvorrichtung direkt im Deckel oder ausserhalb des Deckels in einer Aufnahme sitzt.

Das eingeschlossene Umfeld umfasst das darin vorhandene Gasvolumen und vom Gasvolumen umspülte Oberflächen, nämlich Oberflächen:
- des Deckels;
- der optionalen Aufnahme;
- einer optionalen Dichtung; und
- von optional freiliegenden Wandungsflächen.

Die Prozesskammer ist mit einem Manipulator oder zusätzlich mit einem zweiten Manipulator ausgestattet, an deren schwenkbaren Auslegern jeweils ein mit Werkzeugen bestückter Werkzeugkopf sitzt, wobei:
- die Werkzeuge ausgebildet sind, um die Abdeckung zu öffnen und anschliessend die Artikel aus dem Behältnis durch den freigelegten Durchlass in die Prozesskammer zu transferieren; und
- der eine Manipulator mit dessen Werkzeugen wechselhaft an den Passagen zum Öffnen der Abdeckung und Transfer der Artikel in die Prozesskammer zum Einsatz kommt; oder
- der eine Manipulator und der zweite Manipulator jeweils nur an einer der Passagen oder die beiden Manipulatoren wechselhaft an den Passagen zum Einsatz kommen.

Ein Schneidwerkzeug ist Komponente der Werkzeuge, um für das Öffnen die Abdeckung aufzuschneiden. Vorzugsweise bestehen von der Abdeckung und der Umhüllung zumindest Flächenanteile aus einem semipermeablen Vliesgewebe, wie Tyvek^{®}. Die Abdeckung bewahrt vor dem Entfernen den Innenraum des Behältnisses in sterilem Zustand und ist auf einem den Durchlass des Behältnisses umlaufenden Rand versiegelt, z.B. durch Verkleben oder Verschweissen.

Im Behältnis lagert zur Aufnahme einer Vielzahl von Artikeln ein entnehmbares Nest, welches zusammen mit den Artikeln in die Prozesskammer transferiert wird. Nach Leerung des Nestes von den Artikeln in der Prozesskammer wird dieses geleerte Nest aus der Prozesskammer in ein nächstes oder dasselbe an der betreffenden Passage angedrücktes geleertes Behältnis entsorgt. Alternativ werden die Artikel direkt aus dem im Behältnis verbleibenden Nest in die Prozesskammer transferiert.

Die Dekontaminationsvorrichtung wird vorzugsweise als Strahlungsquelle, z.B. UVC, UV, E-Beam oder Puls-Licht, oder alternativ als eine Begasungseinrichtung, z.B. zur Verneblung einer H₂O₂-Lösung, ausgebildet.

Die von einem Gehäuse umgebene Eingangsstation weist einen zu einer angrenzenden Bereitstellungsstation führenden Zugang auf, wobei:
- die Eingangsstation mit einer gereinigten, gleichgerichteten Verdrängungsströmung betrieben wird, die teilweise durch den Zugang austritt;
- an der Bereitstellungsstation die Gebinde angeliefert werden, welche im Anlieferungszustand jeweils von einer Umhüllung umgeben sind, um das Innenvolumen des Gebindes mit dessen Inhalt aseptisch zu erhalten; und
- an der Bereitstellungsstation eine an der Umhüllung angebrachte Öffnung, die man bis anhin geschlossen hält, nun auf der der aus dem Zugang austretenden Verdrängungsströmung zugewandten Seite freigegeben wird, um das einzelne Gebinde in die Eingangsstation einzubringen; oder
- an der Bereitstellungsstation die Umhüllung auf der der aus dem Zugang austretenden Verdrängungsströmung zugewandten Seite aufgetrennt wird, um das einzelne Gebinde in die Eingangsstation einzubringen.

Die Positionierung des in der zuerst noch geschlossenen Umhüllung steckenden Gebindes erfolgt auf einer Auflage. Zum Ausschieben des Gebindes aus der offenen Umhüllung, bei auf das Gebinde fliessender Verdrängungsströmung, dient ein Vorschubmittel. Die Umhüllung wird von einem Greifer, vorzugsweise als Sauger ausgebildet, gehalten.

Die in die Eingangsstation eingebrachten Gebinde werden mittels zumindest einer Fördereinrichtung den Passagen zugeführt und nach der Verarbeitung über diese Fördereinrichtung wieder abgeführt. Die zumindest eine Fördereinrichtung beruht vorzugsweise auf dem Prinzip der Magnetschwebetechnik und erlaubt die Förderung auf mehreren Spuren, auch in gegensätzlicher Richtung.

Mittels einer ersten Fördereinrichtung erfolgt der Transport der Gebinde innerhalb der Eingangsstation, während eine zweite Fördereinrichtung das Heranschwenken der Gebinde mit an der jeweiligen Passage angepressten Abdeckung bewirkt. Die zweite Fördereinrichtung ist auch zum Entfernen bearbeiteter Gebinde von der jeweiligen Passage nutzbar. Zur Positionierung der Gebinde an der jeweiligen Passage umfasst die zweite Fördereinrichtung vorzugsweise ein kardanisches Lager.

Die zweite Fördereinrichtung besteht aus:
- einer Achse, durch welche sich eine erste Drehachse erstreckt;
- zwei nebeneinander an der Achse fixierten Gabeln, die jeweils um eine zweite Drehachse schwenkbar sind, welche sich rechtwinklig zur ersten Drehachse erstrecken; und
- jeweils einen in der zugehörigen Gabel aufgehängten und um eine dritte Drehachse schwenkbaren Träger, wobei die erste Drehachse und die dritte Drehachse zueinander parallel verlaufen und die Träger zum Unterfassen der Ränder der Behältnisse beim An- bzw. Abschwenken von den Passagen konfiguriert sind.

Zwei nebeneinander liegende kardanische Lager sind jeweils gebildet aus einen U-förmigen Träger, der in der dritten Drehachse jeweils mit einer Gabel verbunden ist, die in der zweiten Drehachse aufgehängt ist.

In jeder Passage sitzt eine Dichtung, welche zusammen mit der Abdeckung eines temporär angepressten Gebindes und/oder mit einem temporär angepressten Deckel an den Passagen, im Übergang von der Eingangsstation zur Prozesskammer, Abdichtungen schafft. Anstelle einer herkömmlichen Dichtung kann man einen minimen Spalt vorsehen oder eine Labyrinth-Dichtung einsetzen. Der Deckel wird von einem schwenkbaren Ausleger einer in der Prozesskammer positionierten Apparatur getragen.

Der zumindest eine Manipulator oder auch der zusätzliche Manipulator sind vorzugsweise als Roboter ausgebildet.

Die Behältnisse der Gebinde haben die Gestalt wannenförmiger Tubs. Die Artikel sind z.B. Phiolen, Vials oder Spritzen, die in systematisch angeordneten Aufnahmekonturen von Nestern stecken.

Das Wesen der Erfindungen beruht auf dem unabhängigen Verfahrensanspruch 1 bzw. auf dem unabhängigen Sachanspruch 18.

### Kurzbeschreibung der beigefügten Zeichnungen

Es zeigen:

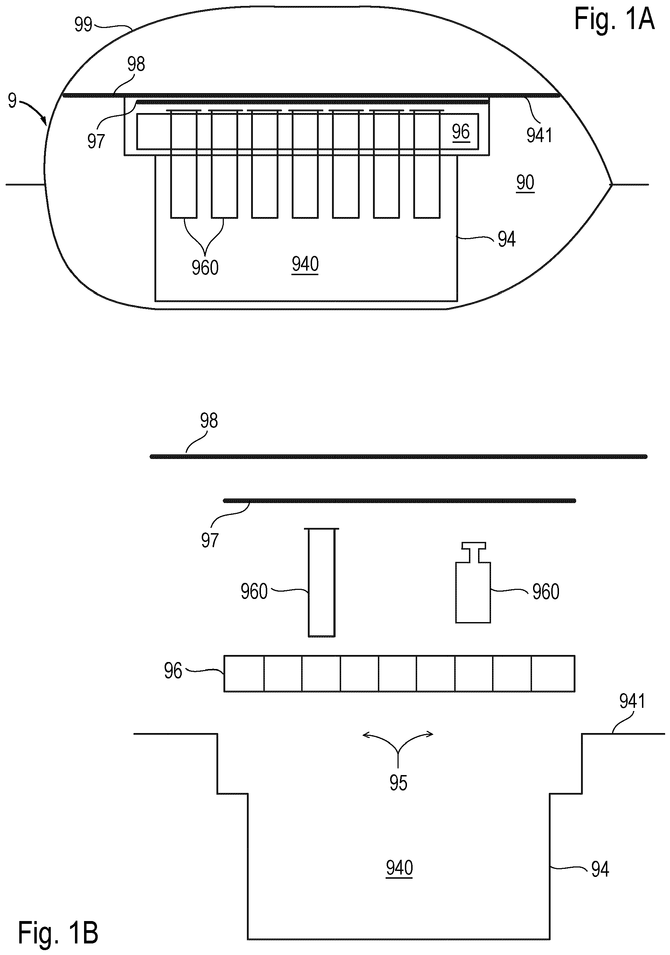

- Figur 1A -: ein mit dem erfindungsgemässen Verfahren in der zugehörigen Schleusenanordnung zu verarbeitendes Gebinde mit geschlossener Umhüllung;
- Figur 1B -: das Gebinde gemäss Figur 1A ohne die Umhüllung in Explosivansicht;

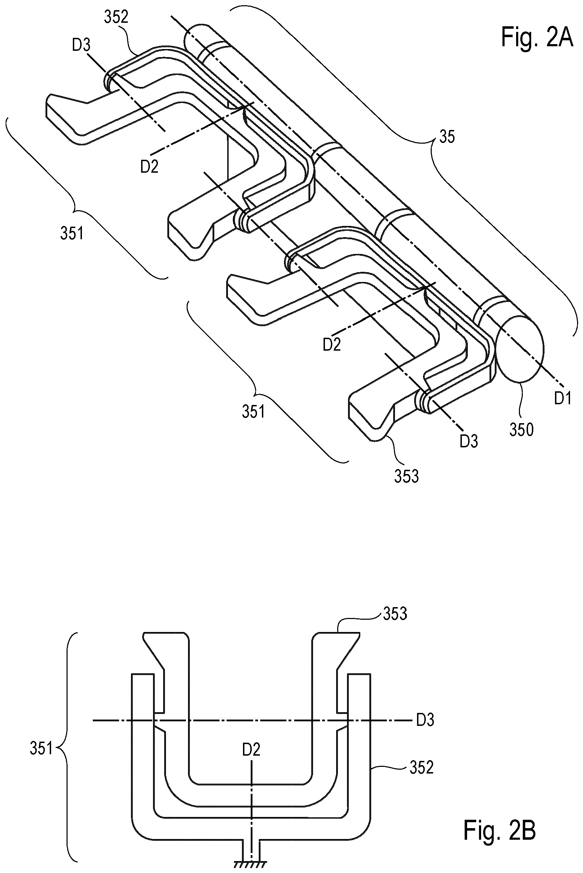

- Figur 2A -: die zweite Fördereinrichtung als Bestandteil der Schleusenanordnung, bestehend aus der Achse und daran fixierten zwei kardanischen Lagern, in Perspektivansicht;
- Figur 2B -: ein kardanisches Lager aus Figur 2A, in Draufsicht;

Figuren 3A bis 15D: die Gesamtheit eines Verfahrensdurchlaufs mit jeweils vier Figuren der Schleusenanordnung, nämlich deren vordere Sektion gemäss Figuren A, hintere Sektion gemäss Figuren B, in Draufsicht gemäss Figuren C und als prinzipielle Seitenansicht gemäss Figuren D;

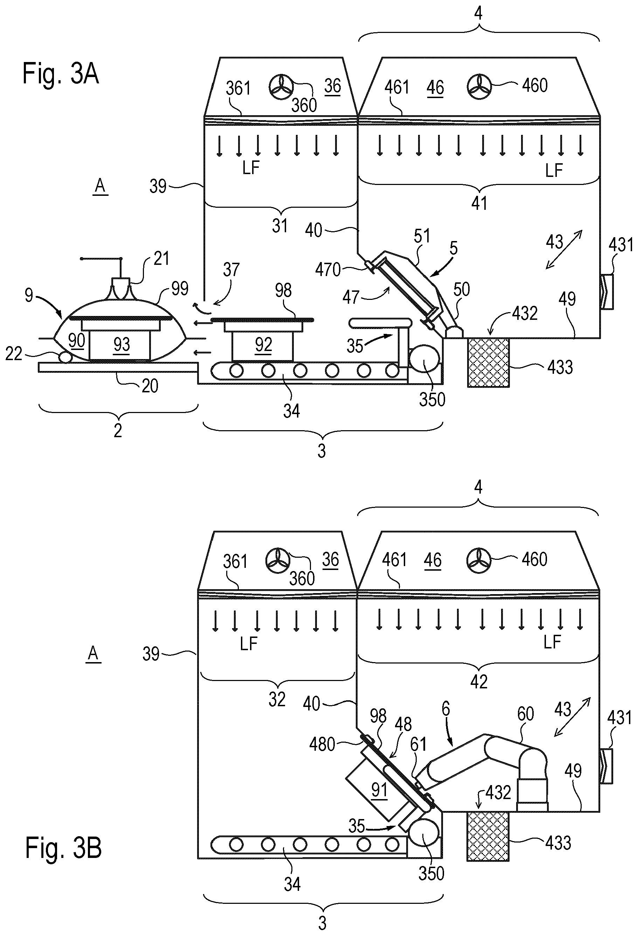

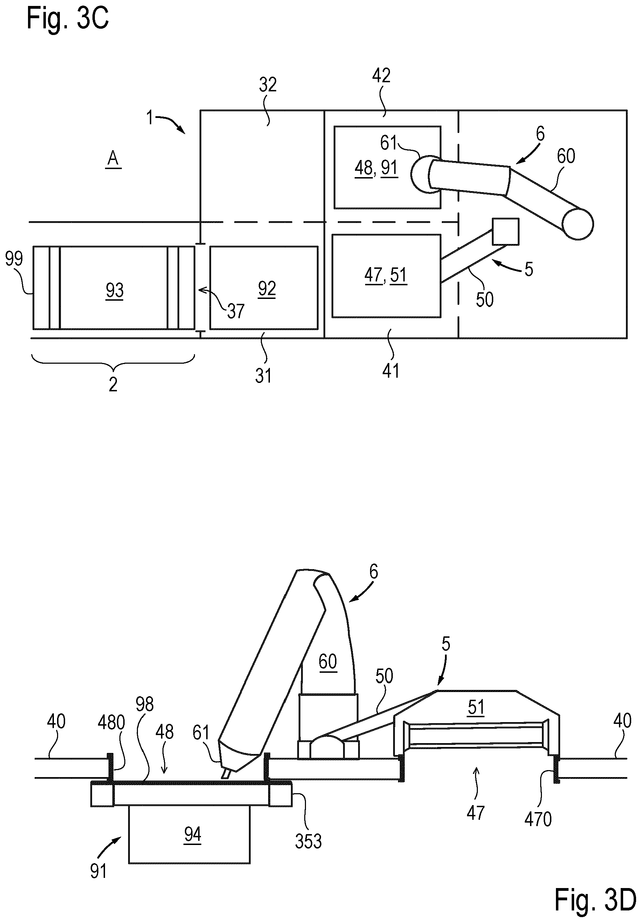

- Figuren 3A bis 3D -: Prozessphase 1 (Startposition)

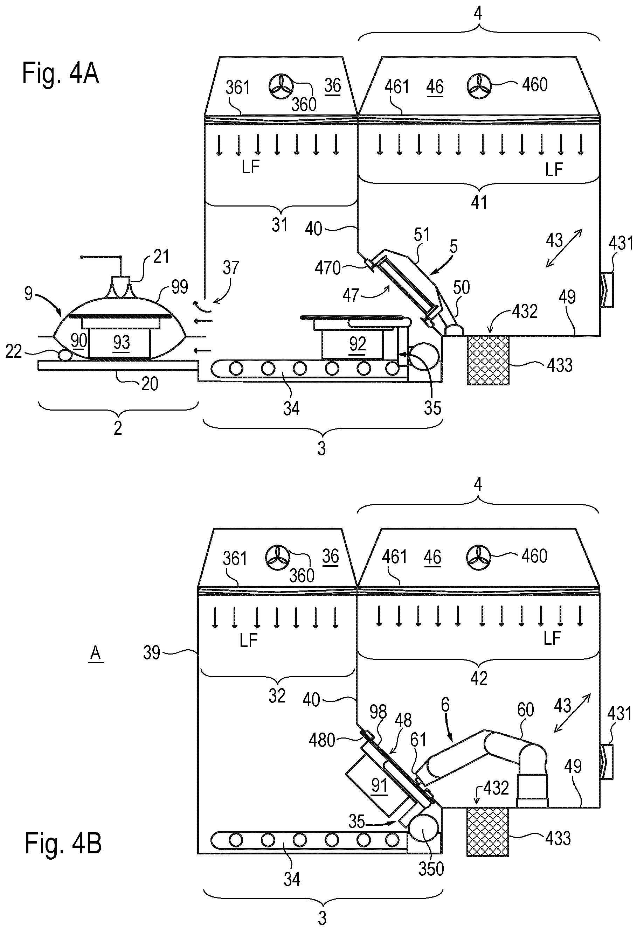

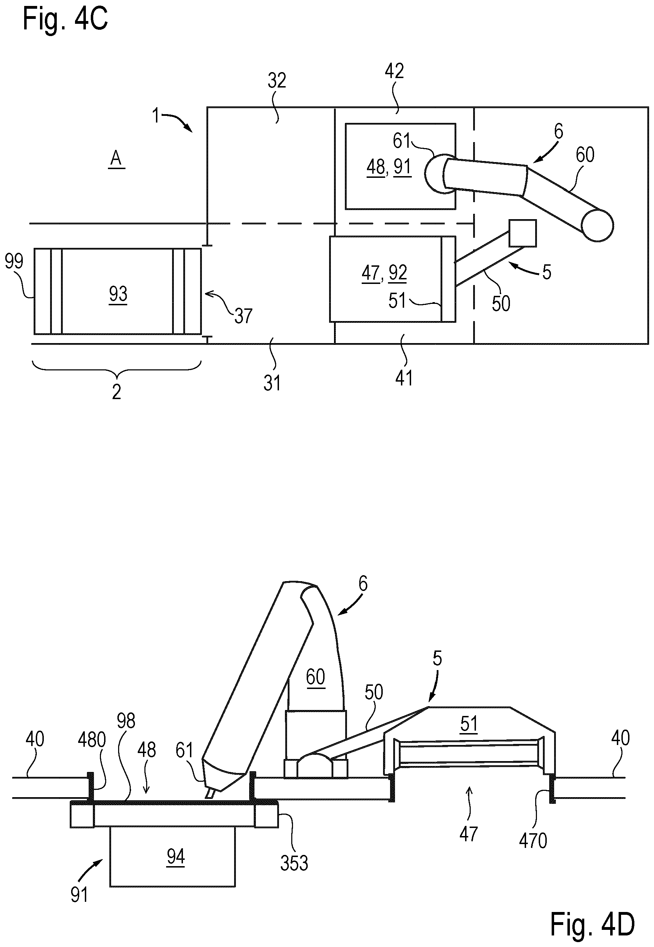

- Figuren 4A bis 4D -: Prozessphase 2

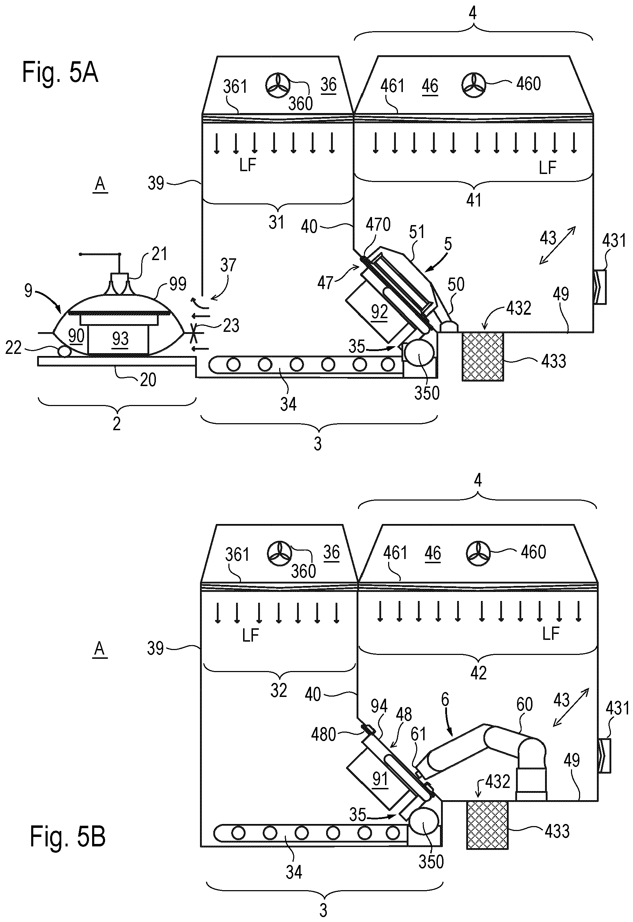

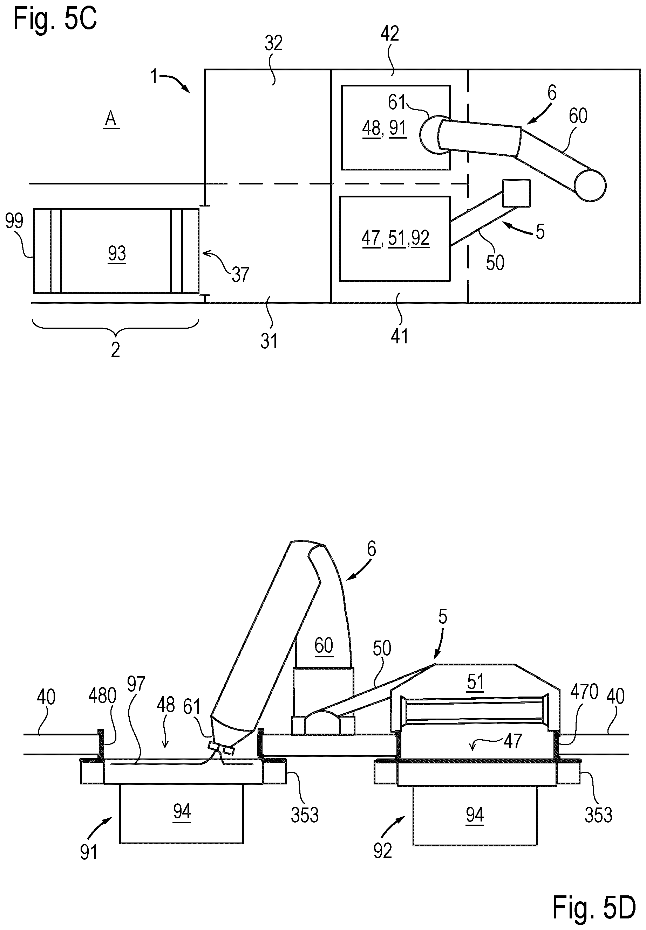

- Figuren 5A bis 5D -: Prozessphase 3

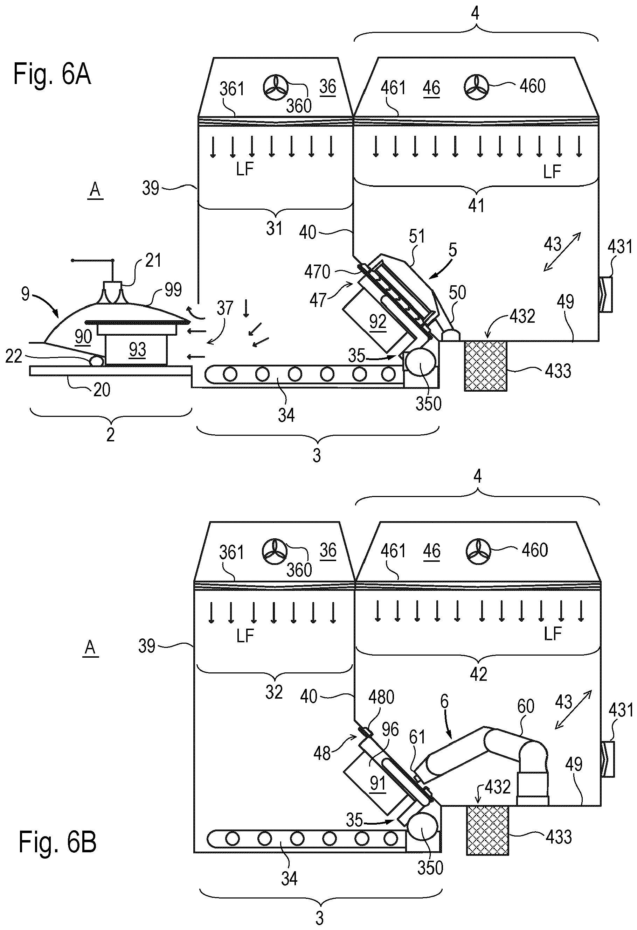

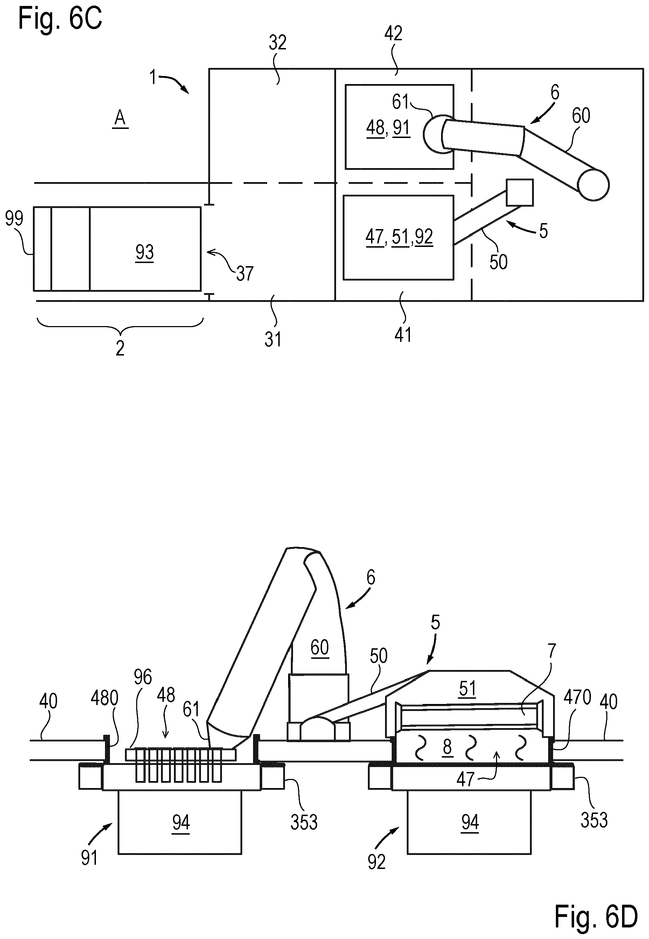

- Figuren 6A bis 6D -: Prozessphase 4

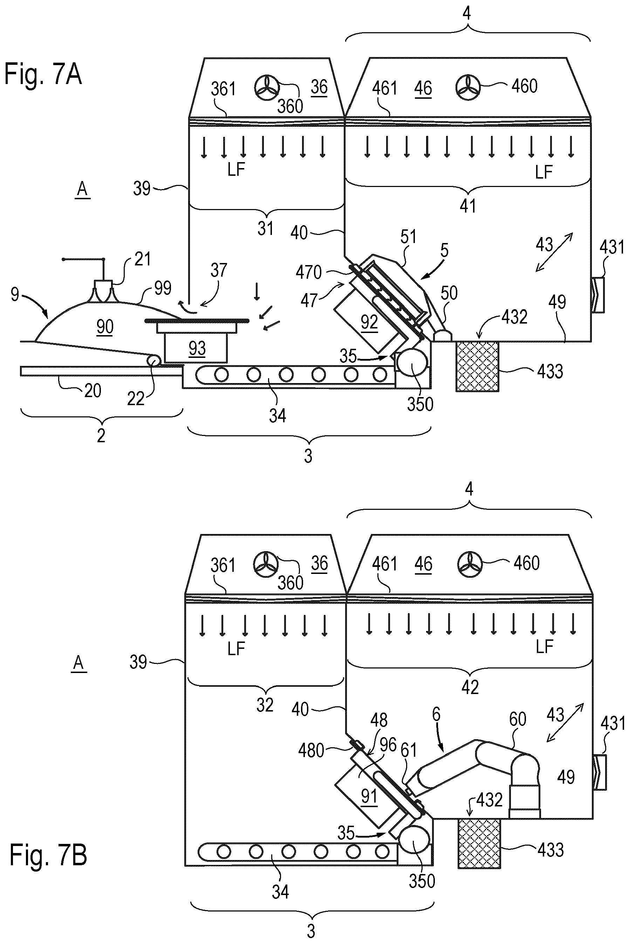

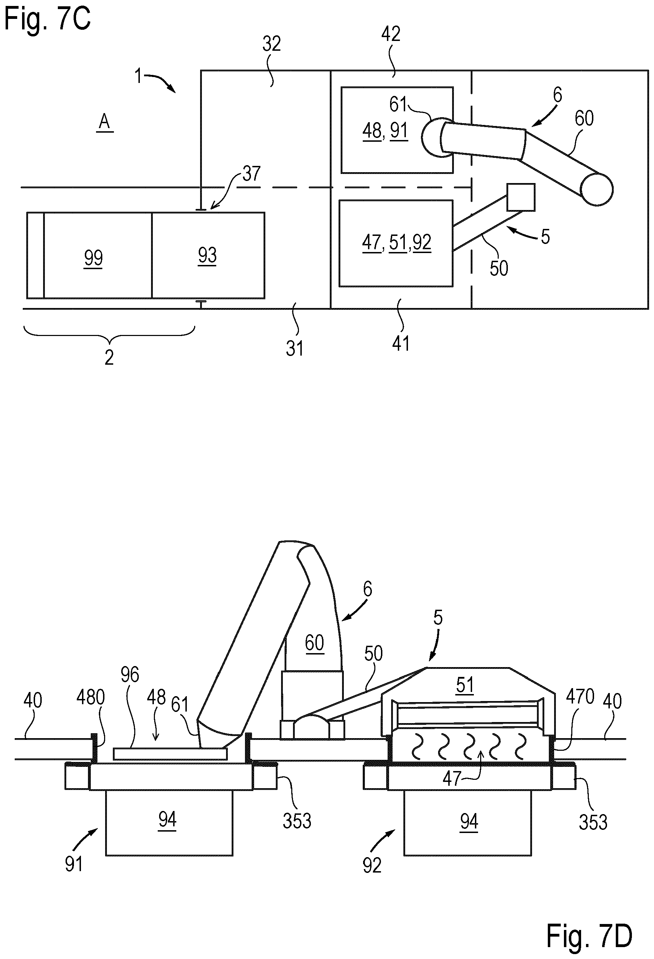

- Figuren 7A bis 7D -: Prozessphase 5

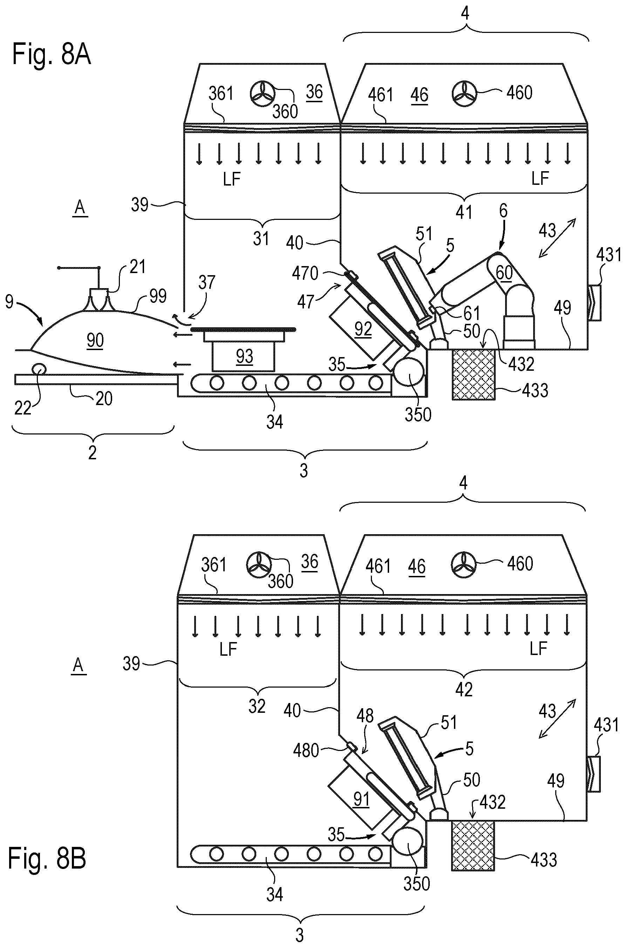

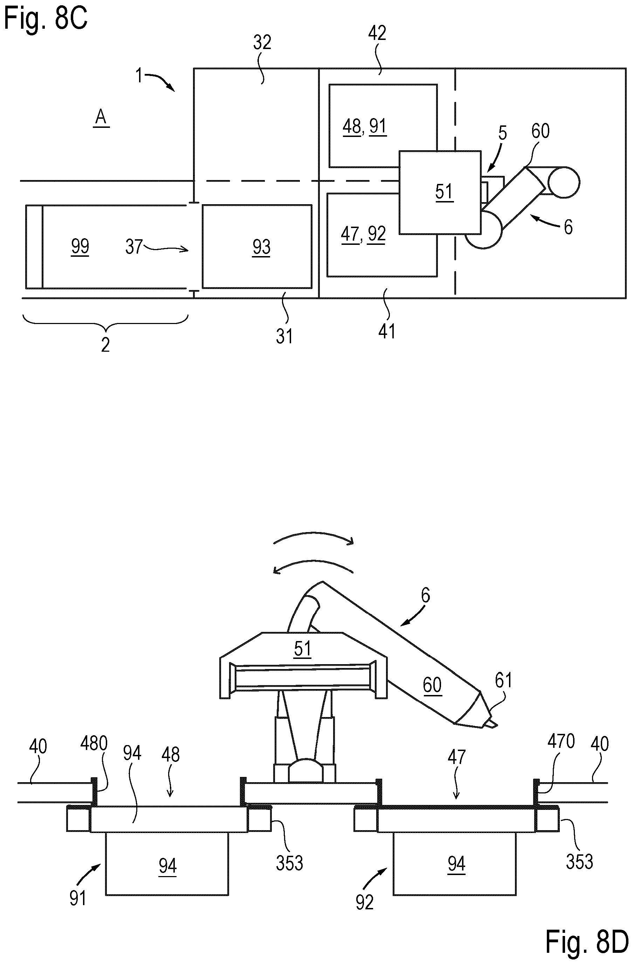

- Figuren 8A bis 8D -: Prozessphase 6

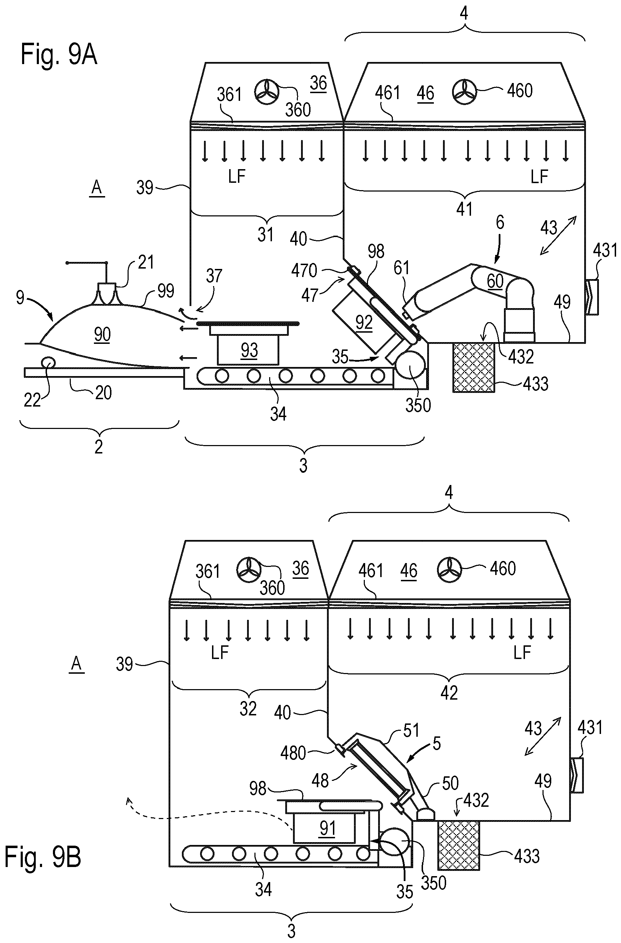

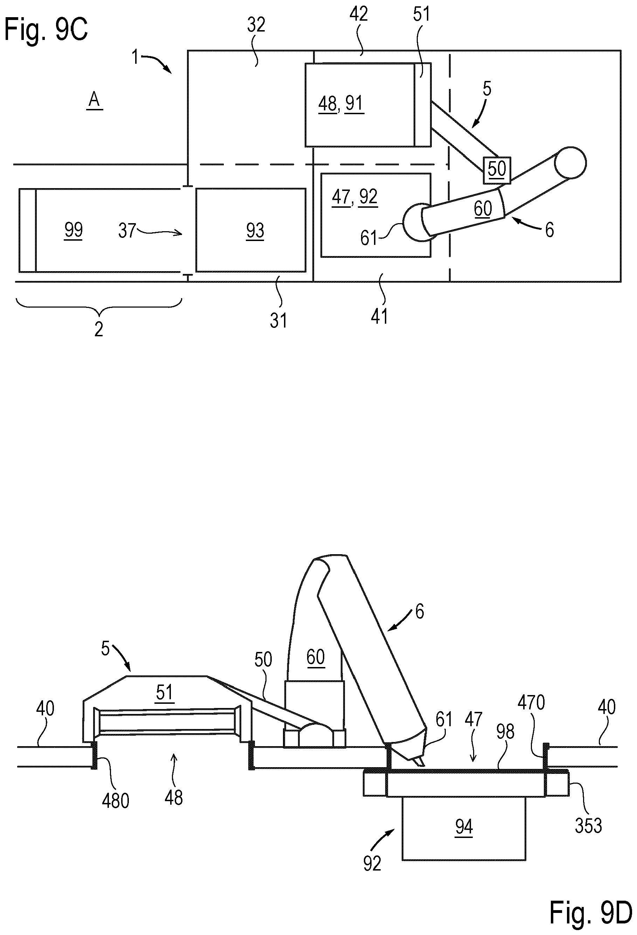

- Figuren 9A bis 9D -: Prozessphase 7

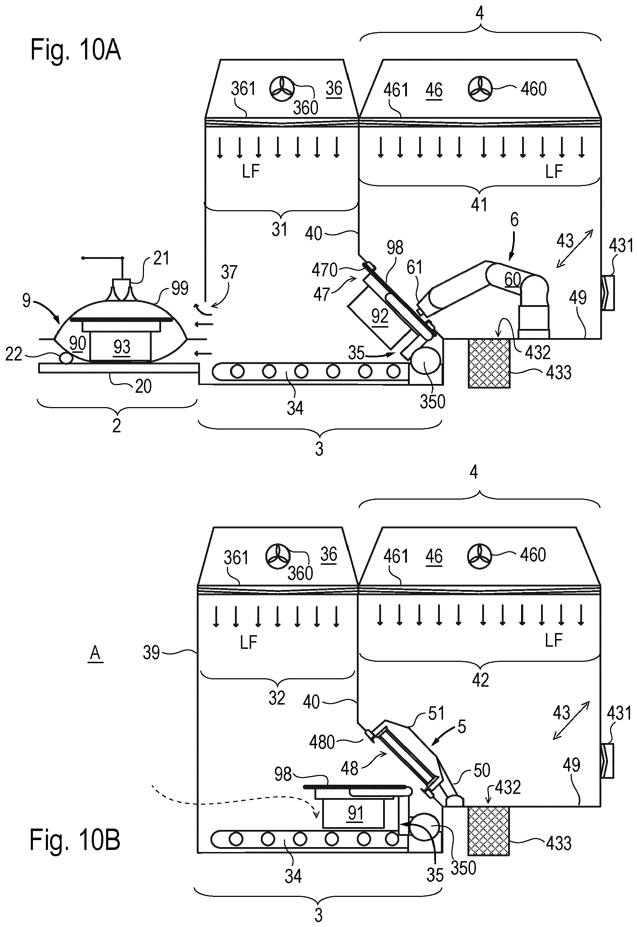

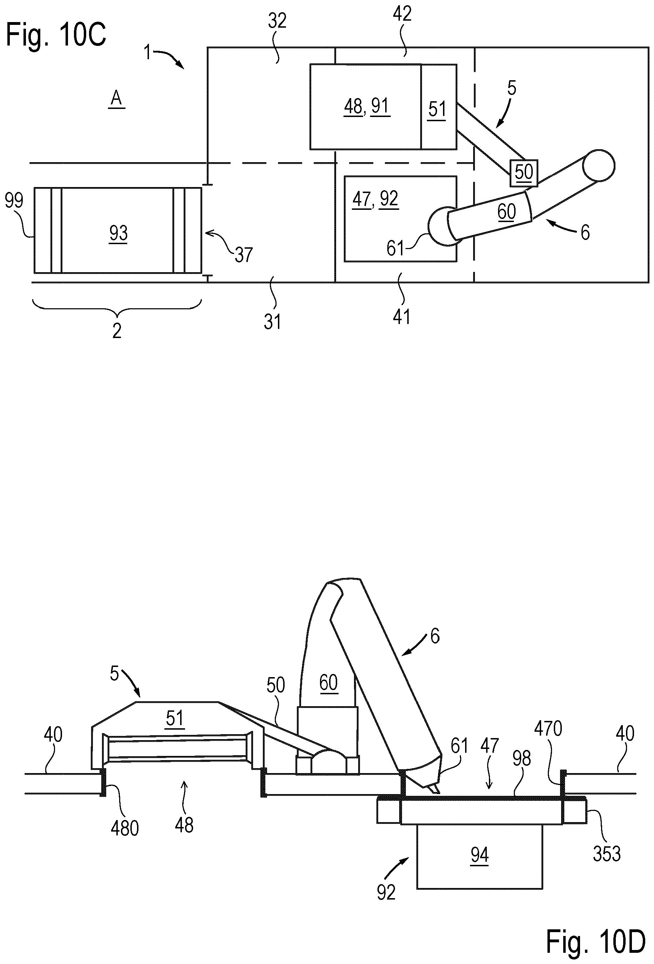

- Figuren 10A bis 10D -: Prozessphase 8

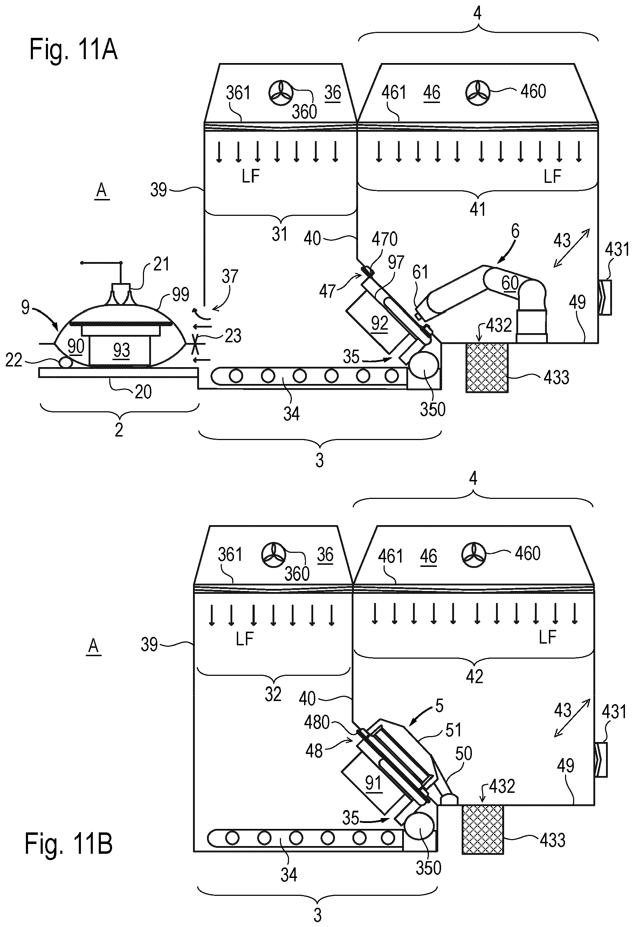

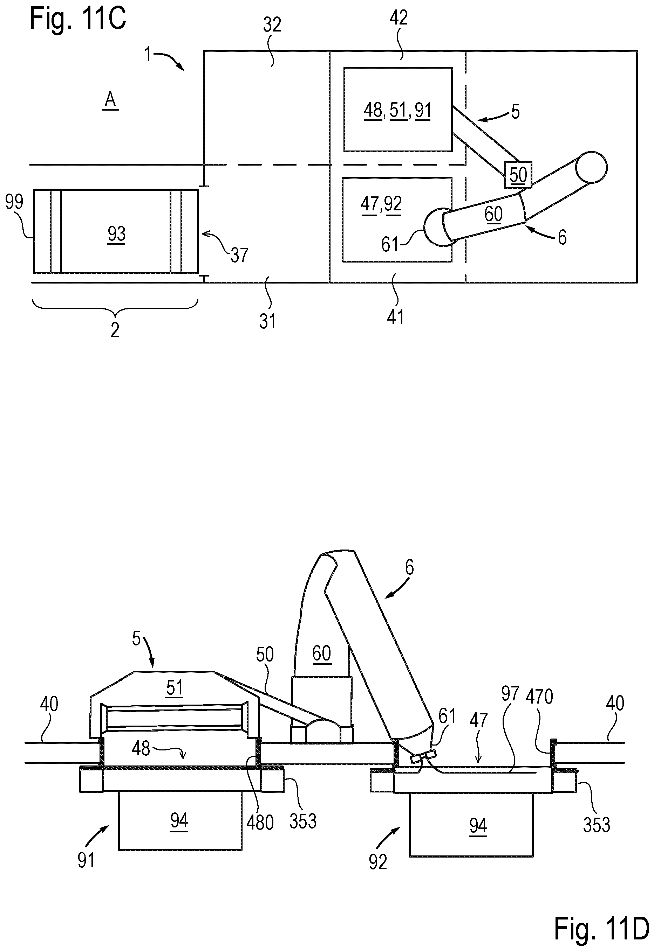

- Figuren 11A bis 11D -: Prozessphase 9

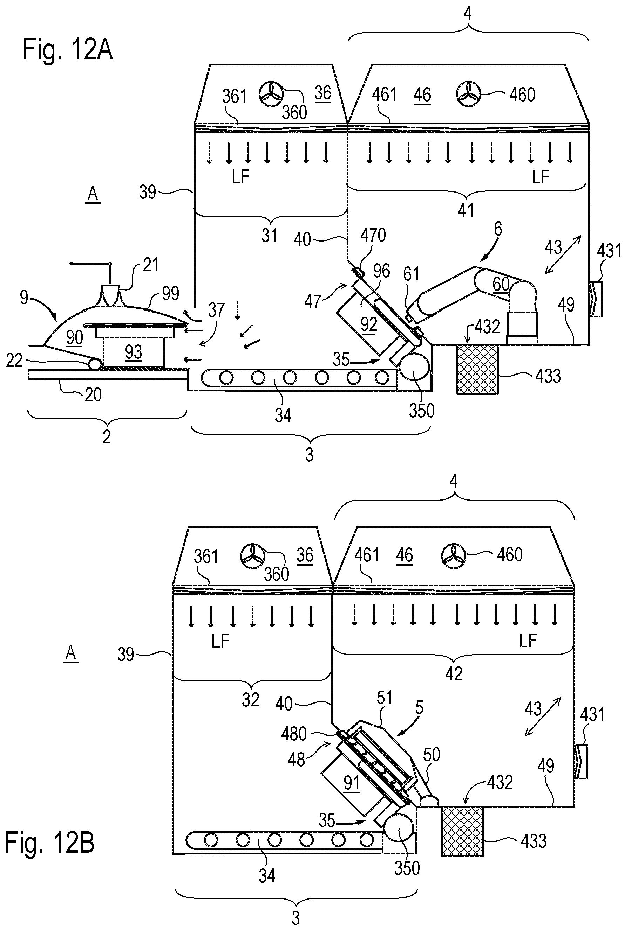

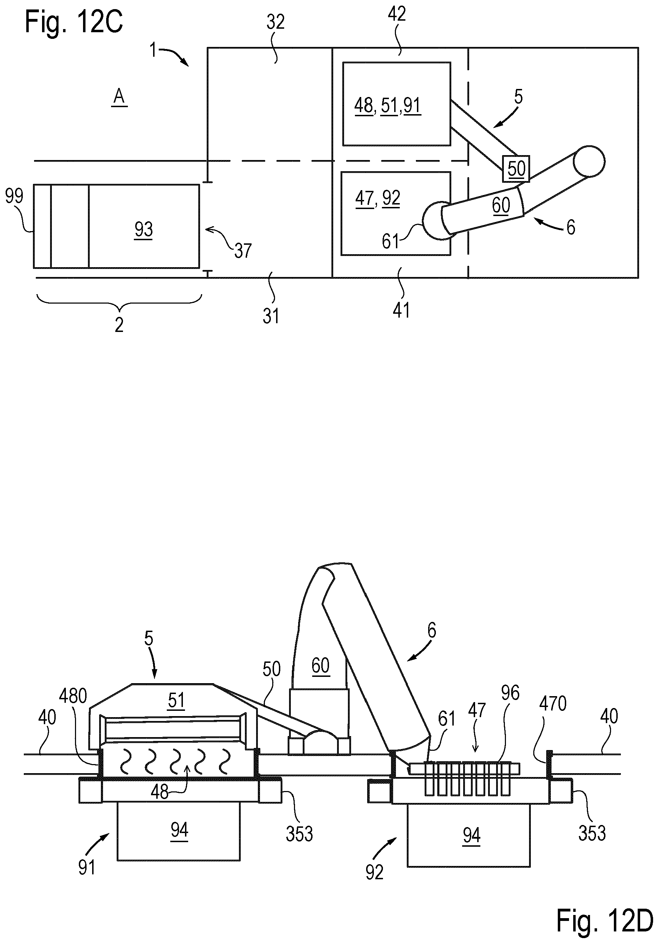

- Figuren 12A bis 12D -: Prozessphase 10

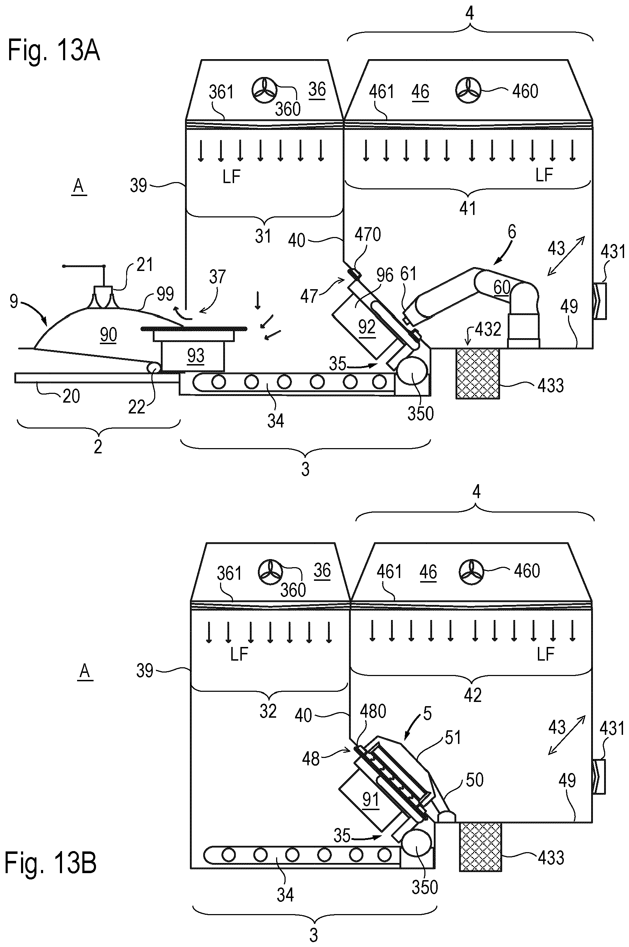

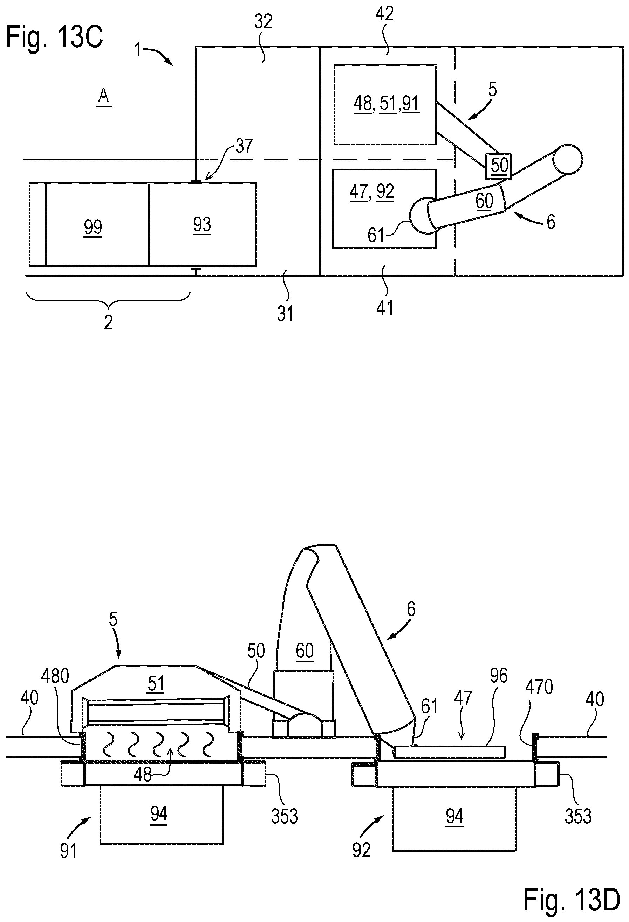

- Figuren 13A bis 13D -: Prozessphase 11

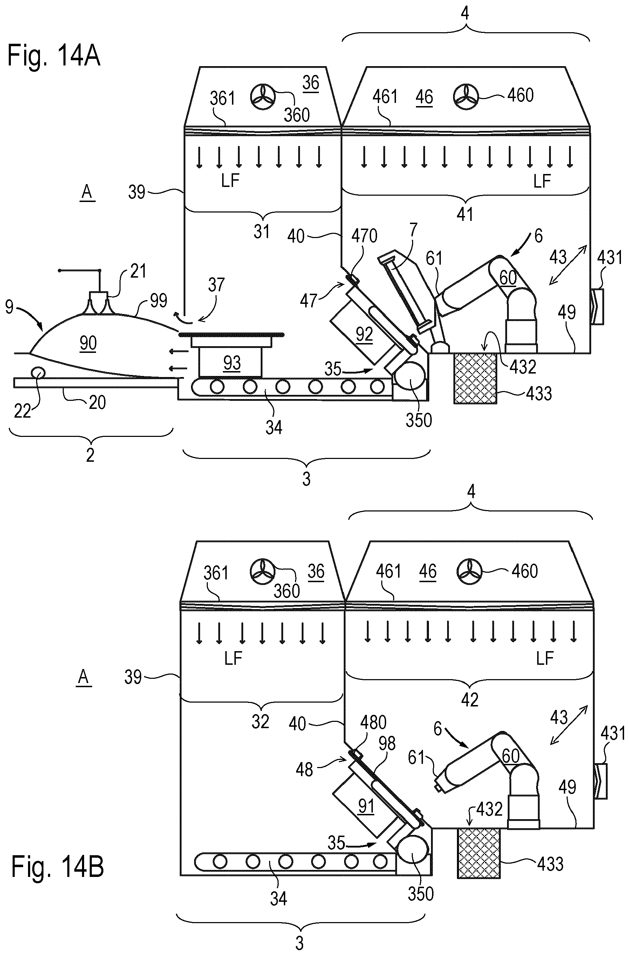

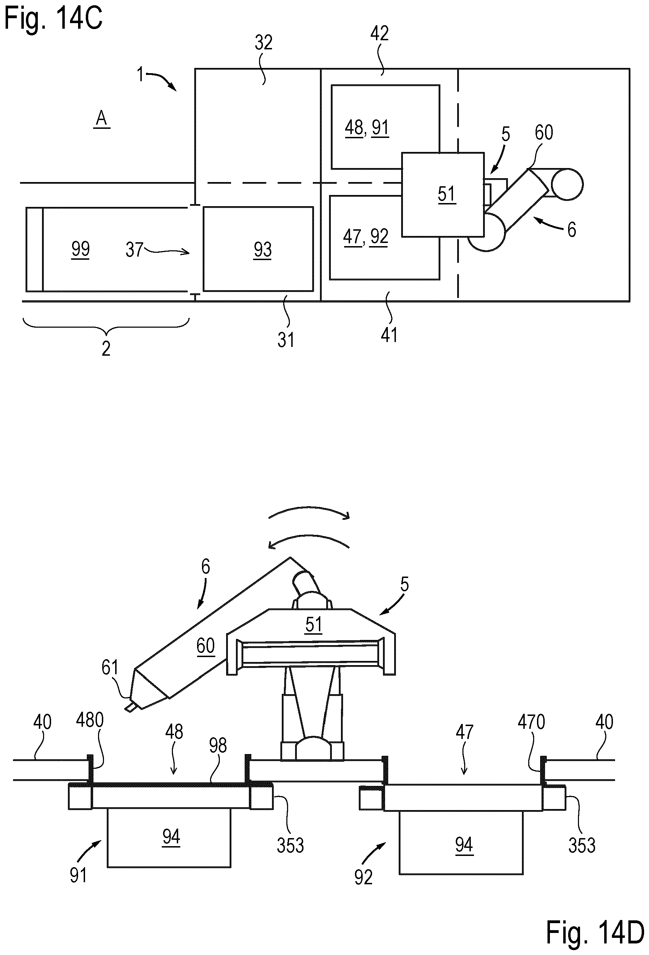

- Figuren 14A bis 14D -: Prozessphase 12

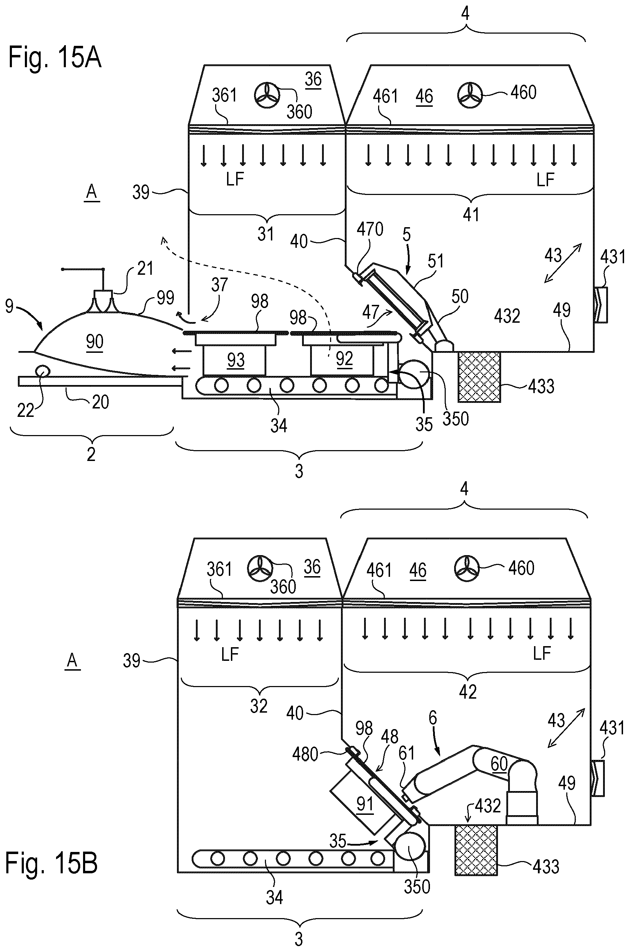

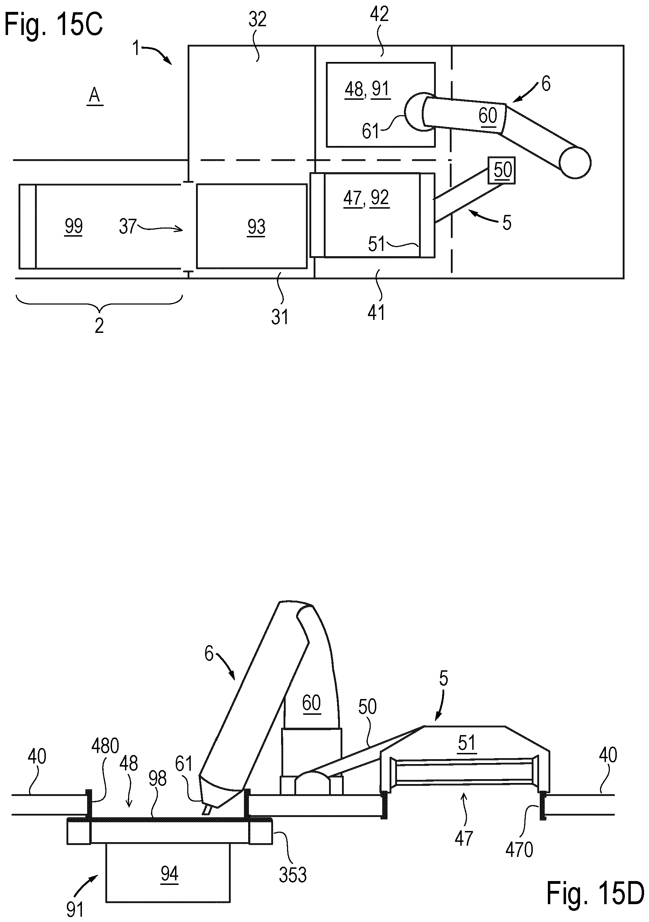

- Figuren 15A bis 15D -: Prozessphase 13

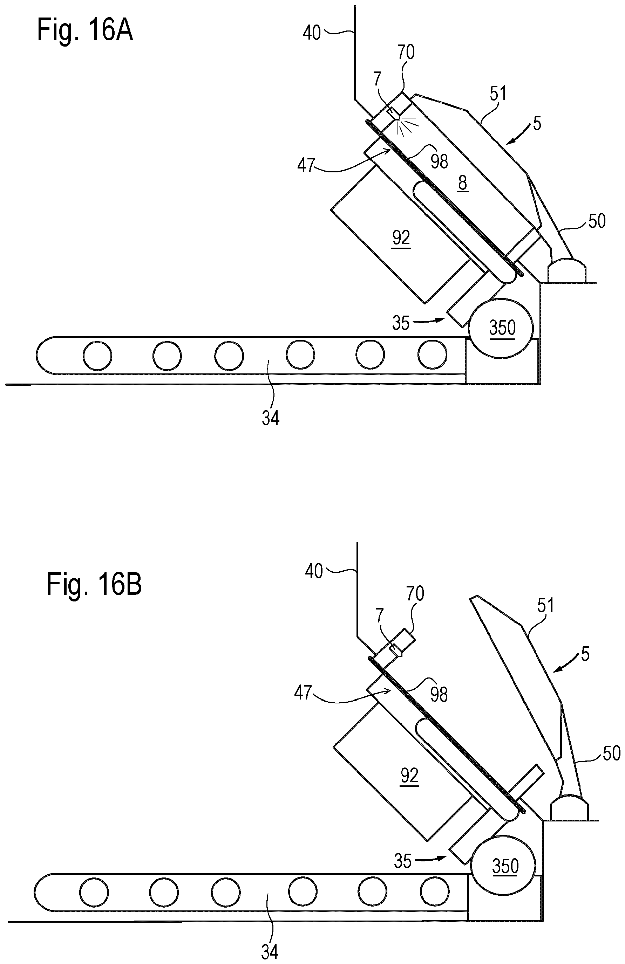

- Figur 16A -: eine alternative Anordnung mit der Dekantaminationsvorrichtung ausserhalb des Deckels, in einer Halterung sitzend, Gebinde an der Passage mittels der hochgeschwenkten zweiten Fördereinrichtung angedrückt, Deckel geschlossen, Dekantaminationsvorrichtung aktiviert; und
- Figur 16B -: die Anordnung gemäss Figur 16A mit geöffnetem Deckel und inaktiver Dekantaminationsvorrichtung.

### Ausführungsbeispiel

Mit Bezug auf die beiliegenden Zeichnungen erfolgt nachstehend die detaillierte Beschreibung des erfindungsgemässen Verfahrens und der erfindungsgemässen Schleusenanordnung zum Einschleusen von Artikeln aus einem Gebinde in die Prozesskammer eines Containments unter aseptischen Bedingungen. Wie die beigefügten Zeichnungen, betrifft die nachfolgend beschriebene Ausführungsform den Transfer von Artikeln aus Gebinden in die Prozesskammer durch zwei von der Eingangsstation in die Prozesskammer führende Passagen. Jede der Passagen lässt sich abwechselnd von einem in der Prozesskammer herangeführten Deckel verschliessen, in welchem das Emissionselement einer Dekontaminationsvorrichtung integriert sein kann.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

### Figuren 1A und 1B

Ein einzelnes Gebinde **9** umfasst:
- ein Behältnis **94** mit einem aseptischen Innenraum **940** und einem Durchlass **95,** der mit einer Abdeckung **98** verschlossen ist;
- ein im Innenraum **940** gelagertes Nest **96,** in dessen muldenförmigen Aufnahmekonturen die Artikel **960** stecken, welche nach Öffnen der Abdeckung **98** in der Prozesskammer **43** des Containments **4** zu behandeln sind;
- optional eine zwischen der Abdeckung **98** und dem Nest **96,** über den Artikeln **960** eingefügte Zwischenlage **97;** sowie
- eine im Anlieferungszustand das gesamte Gebinde **9** umgebende, geschlossene Umhüllung **99,** die das Innenvolumen **90** steril hält.

Das Behältnis **94** ist z.B. ein wannenförmiges Tub. Die Artikel **960** sind insbesondere Phiolen, Vials oder Spritzen. Von der Abdeckung **98** und der Umhüllung **99** bestehen vorzugsweise zumindest Flächenanteile aus einem semipermeablen Vliesgewebe, wie Tyvek^{®}. Die Abdeckung **98** ist typischerweise auf dem den Durchlass **95** des Behältnisses **94** umlaufenden Rand **941** versiegelt und bewahrt so den Innenraum **940** des Behältnisses **94** in sterilem Zustand.

### Figuren 2A und 2B

Als Bestandteil der Schleusenanordnung **1** sind die frontseitige erste Zone **31** und die rückseitige zweite Zone **32** der Eingangsstation **3** jeweils mit einer zweiten Fördereinrichtung **35** ausgestattet. Eine solche Fördereinrichtung **35** besteht aus einer Achse **350** und daran fixierten zwei kardanischen Lagern **351.** Durch die Achse **350** erstreckt sich eine erste Drehachse **D1.** Zwei nebeneinander an der Achse **350** fixierte Gabeln **352** sind jeweils um eine zweite Drehachse **D2** schwenkbar, welche sich rechtwinklig zur ersten Drehachse **D1** erstrecken. In jeder Gabel **352** ist ein um eine dritte Drehachse **D3** schwenkbarer Träger **353** aufgehängt, wobei die erste Drehachse **D1** und die dritte Drehachse **D3** zueinander parallel verlaufen. Die Träger **353** sind zum Unterfassen der Ränder **941** der Behältnisse **94** beim An- bzw. Abschwenken von den von der Eingangsstation **3** in die Prozesskammer **43** führenden Passagen **47,48** konfiguriert.

### Figuren 3A bis 3D

### Aufbau der Schleusenanordnung 1

Die im Aufstellraum **A** installierte Schleusenanordnung **1** strukturiert sich in eine Eingangsstation **3,** eine dieser vorgelagerte Bereitstellungsstation **2** und das sich an die Eingangsstation **3** anschliessende Containment **4** mit dessen Prozesskammer **43.** Die Bereitstellungsstation **2** besitzt die Auflage **20,** um darauf neue, dritte Gebinde **93** mit zunächst noch mit verschlossener Umhüllung **99** zu positionieren. Das Vorschubmittel **22** dient dem Ausschieben solcher dritten Gebinde **93** aus der geöffneten Umhüllung **99** von der Auflage **20** durch den Zugang **37** im die Eingangsstation **3** umgebenden Gehäuse **39** auf die erste Fördereinrichtung **34** in der frontseitigen ersten Zone **31** der Eingangsstation **3.** Während des Ausschiebens hält der in der Bereitstellungsstation **2** vorhandene Greifer **21** die Umhüllung **99** zurück. An die erste Fördereinrichtung **34** schliesst sich die zweite Fördereinrichtung **35** an. Im Plenum **36** der frontseitigen ersten Zone **31** der Eingangsstation **3** sind ein Zuluftventilator **360** sowie Zuluftfilter **361** zur Erzeugung eines gereinigten, abwärts laminar strömenden Luftstromes **LF** angeordnet.

Zwischen der frontseitigen ersten Zone **31** der Eingangsstation **3** und der frontseitigen ersten Zone **41** der Prozesskammer **43** erstreckt sich die Trennwand **40,** welche die erste Passage **47** zwischen den Zonen **31,41** aufweist. In der ersten Passage **47** sitzt die Dichtung **470.** Auch im Plenum **46** der frontseitigen ersten Zone **41** des Containments **4** sitzen ein Zuluftventilator **460** und ein Zuluftfilter **461** zur Erzeugung eines gereinigten, abwärts laminar strömenden Luftstromes **LF.** Im die Prozesskammer **43** umgebenden Gehäuse **49** sind seitlich und/oder alternativ in dessen Boden ein Abluftfilter **431** und im Boden eine Abfallöffnung **432** vorgesehen, die zu einem Depot **433** führt.

In der sich über die frontseitige erste Zone **41** und die rückseitige zweite Zone **42** erstreckende gemeinsame Prozesskammer **43** ist eine Apparatur **5** mit dem Ausleger **50** und dem daran gehaltenen Deckel **51** stationiert, in dem die Dekontaminationsvorrichtung **7** integriert ist. Durch Schwenken des Auslegers **50** kann der Deckel **51** über Zwischenstellungen an die erste Passage **47** oder die zweite Passage **48** herangeführt werden.

In der Eingangsstation **3** liegt hinter der ersten Zone **31** die rückseitige zweite Zone **32,** und im Containment **4** liegt hinter der ersten Zone **41** die rückseitige zweite Zone **42.** Äquivalent zur ersten Zone **31** ist die zweite Zone **32** ebenfalls in deren Plenum **36** mit einem Zuluftventilator **360** und einem Zuluftfilter **361** zur Erzeugung eines Luftstromes **LF** ausgestattet. Wiederum vorhanden sind eine erste Fördereinrichtung **34** und die sich daran anschliessende zweite Fördereinrichtung **35** an. Gleichfalls äquivalent zur ersten Zone **41** hat die zweite Zone **42** das Plenum **46** mit Zuluftventilator **460** und Zuluftfilter **461** zur Erzeugung des Luftstromes **LF.**

Bei kleineren Anlagen können ein gemeinsamer Zuluftventilator **360** und ein gemeinsamer Zuluftfilter **361** für die erste Zone **31** und die zweite Zone **32** sowie ein gemeinsamer Zuluftventilator **460** und ein gemeinsamer Zuluftfilter **461** für die erste Zone **41** und die zweite Zone **42** genügen. Bei grösseren Anlagen wird man für die Zonen **31,32** bzw. **41,42** mehrere Zuluftventilatoren **360,460** bzw. Zuluftfilter **361,461** vorsehen. In der rückseitigen zweiten Zone **42** sind ebenfalls im Gehäuse **49** seitlich und/oder alternativ in dessen Boden der Abluftfilter **431** und im Boden die zu einem Depot **433** führende Abfallöffnung **432** vorhanden.

Zwischen der rückseitigen zweiten Zone **32** der Eingangsstation **3** und der rückseitigen zweiten Zone **42** der Prozesskammer **43** erstreckt sich erneut die Trennwand **40,** welche nun die zweite Passage **48** zwischen den Zonen **32,42** aufweist. In dieser zweiten Passage **48** sitzt die Dichtung **480.** In der für die erste und zweite Zone **41,42** gemeinsamen Prozesskammer **43** ist ein Manipulator **6** mit dem Ausleger **60** und dem daran befindlichen Werkzeugkopf **61** stationiert. Durch Schwenken des Auslegers **60** kann der Werkzeugkopf **61** über Zwischenstellungen an die erste Passage **47** oder die zweite Passage **48** herangeführt werden.

### Ablauf in Prozessphase 1 (Startposition)

- In Bereitstellungsstation **2:**
   Ein neues, drittes Gebinde **93,** noch mit verschlossener Umhüllung **99,** ist auf der Auflage **20** angeliefert, befindet sich vor dem Zugang **37** und wird von durch diesen ausfliessender Strömung **LF** angeströmt.
- In Eingangsstation **3,** in deren frontseitiger erster Zone **31:**
   Ein zweites Gebinde **92,** bereits aus der Umhüllung **99** befreit, befindet sich auf der ersten Fördereinrichtung **34.** Vonseiten der ersten Zone **31** ist die erste Passage **47** quasi offen.
- In Eingangsstation **3,** rückseitige zweite Zone **32:**
   Die zweite Passage **48** in die Prozesskammer **43** des Containments **4** ist vom angedrückten ersten Gebinde **91** mit dessen Abdeckung **98** verschlossen, jedoch bereits dekontaminiert.
- Im Containment **4,** frontseitige erste Zone **41:**
   Die erste Passage **47** in die Prozesskammer **43** ist vom Deckel **51** mit der darin integrierten Dekontaminationsvorrichtung **7** verschlossen. Der Deckel **51** wurde von der Apparatur **5** mit dem geschwenkten Ausleger **50** aus der Prozesskammer **43** heraus herangeführt.
- Im Containment **4,** rückseitige zweite Zone **42:**
   Mit einem Werkzeug, welches am Werkzeugkopf **61** eines geschwenkten Auslegers **60** des in der Prozesskammer **43** stationierten Manipulators **6** montiert ist, wird die Abdeckung **98** geöffnet, z.B. aufgeschnitten, und entfernt.

Bei der nachstehenden Beschreibung des Ablaufs in den weiteren Prozessphasen 2 bis 13 (Figuren 4A bis 15D) werden zur Vermeidung von Wiederholungen jeweils nur die Veränderungen zur vorherigen Prozessphase aufgelistet.

### Ablauf in Prozessphase 2 (Figuren 4A bis 4D)

- In Eingangsstation **3,** frontseitige erste Zone **31:**
   Das zweite Gebinde **92,** ohne Umhüllung **99,** noch auf der ersten Fördereinrichtung **34** stehend, wird von der zweiten Fördereinrichtung **35** übernommen.

### Ablauf in Prozessphase 3 (Figuren 5A bis 5D)

- In Bereitstellungsstation **2:**
   Die bis anhin verschlossene Umhüllung **99** um das neue, dritte Gebinde **93** wird mittels des Werkzeugs **23** geöffnet, z.B. aufgeschnitten; hierbei hält der Greifer **21** die Umhüllung **99.** Alternativ kann ein zuvor an der Umhüllung **99** angebrachter Schnitt, den man zunächst, z.B. zuklemmt, jetzt freigegeben werden.
- In Eingangsstation **3,** frontseitige erste Zone **31:**
   Von der zweiten Fördereinrichtung **35** wurde das zweite Gebinde **92** an die zweite Passage **48** herangeführt, welche nun beidseitig vom zweiten angedrückten Gebinde **92** bzw. aufgesetzten Deckel **51** verschlossen ist.
- Im Containment **4,** rückseitige zweite Zone 42:
   Mit einem Werkzeug, das am Werkzeugkopf **61** des schwenkbaren Auslegers **60** des Manipulators 6 installiert ist, wird bei offener Abdeckung **98** die optional vorhandene Zwischenlage **97** aus dem ersten Gebinde **91** entnommen.

### Ablauf in Prozessphase 4 (Figuren 6A bis 6D)

- In Bereitstellungsstation **2:**
   Das neue, dritte Gebinde **93** wird aus der aufseiten des Zugangs **37** offenen Umhüllung **99** vom bewegten Vorschubmittel **22** sukzessive in Richtung Zugang **37** ausgeschoben und dabei von der gereinigten Strömung **LF** bestrahlt. Dies verhindert das Umspülen des Gebindes **93** mit viel unreiner Umgebungsluft aus dem Aufstellraum **A.** Vom Greifer **21** wird die Umhüllung **99** festgehalten.
- Im Containment **4,** frontseitige erste Zone **41:**
   Die Dekontaminationsvorrichtung **7** wird aktiviert, um das zwischen Deckel **51,** Abdeckung **98** auf erstem Gebinde **91,** der die erste Passage **47** umrandende Dichtung **470** und möglichen freiliegenden Wandungsflächen im Umfeld **8** vorhandene Gasvolumen und alle davon umspülten Oberflächen zu dekontaminieren.
- Im Containment **4,** rückseitige zweite Zone **42:**
   Mit einem vom Manipulator **6** herangeführten Werkzeug werden aus dem Innenvolumen **90** aus dem Behältnis **94** des ersten Gebindes **91** das Nest **96,** zusammen mit den darin gelagerten Artikeln **960** oder nur die Artikel **960,** in die Prozesskammer **43** zur weiteren Verarbeitung transferiert.

### Ablauf in Prozessphase 5 (Figuren 7A bis 7D)

- In Bereitstellungsstation **2** und Eingangsstation **3,** frontseitige erste Zone **31:**
   Das neue, dritte Gebinde **93** wird durch pushende Bewegung des Vorschubmittels **22** aus der Umhüllung **99** heraus, durch den Zugang **37** im Gehäuse **39** und gegen die gereinigte Strömung **LF,** auf die erste Fördereinrichtung **34** in die erste Zone **31** der Eingangsstation **3** geschoben. Das Ausschieben des dritten Gebindes **93** aus der Umhüllung **99** geschieht direkt auf die erste Fördereinrichtung **34,** um eine Berührung zwischen Gebinde **93** und Auflage **20** zu vermeiden und so keine Übertragung von Verunreinigungen zu erlauben. Der Greifer **21** hält dabei die Umhüllung **99** zwecks Entsorgung zurück.
- Im Containment **4,** rückseitige zweite Zone **42:**
   In das in vorheriger Prozessphase geleerte Behältnis **94** wird ein aus früherem oder gegenwärtigem Produktionszyklus aus der Prozesskammer **43** stammendes Nest **96** mittels des Manipulators **6** in dieses Behältnis **94** rückgeführt.

### Ablauf in Prozessphase 6 (Figuren 8A bis 8D)

- In Bereitstellungsstation **2** und Eingangsstation **3,** frontseitige erste Zone **31:**
   Das neue, dritte Gebinde **93** ist komplett in die erste Zone **31** eingeschoben und wird von erster Fördereinrichtung **34** getragen. Auf der Auflage **20** verbleiben die vom Greifer **21** zurückgehaltene und zur Entsorgung bestimmte Umhüllung **99** sowie das in Ausgangsposition zurückbewegte Vorschubmittel **22.**
- Im Containment **4,** frontseitige erste Zone **41:**
   Die im Deckel **51** integrierte Dekontaminationsvorrichtung **7** kann abgeschaltet werden; die Behandlung ist abgeschlossen. Mit Abschwenken des Auslegers **50** der Apparatur **5** wird der Deckel **51** von der ersten Passage **47** abgehoben. Zugleich wird der Werkzeugkopf **61** des Manipulators **6** hin zur ersten Passage **47** bewegt.
- Im Containment **4,** rückseitige zweite Zone **42:**
   Mit Umschwenken des Auslegers **50** der Apparatur **5** wird der Deckel **51** der zweiten Passage **48** angenähert.

### Ablauf in Prozessphase 7 (Figuren 9A bis 9D)

- Im Containment **4,** frontseitige erste Zone **41:**
   Der Deckel **51** ist von der ersten Passage **47** entfernt und das vom Manipulator **6** geführte Werkzeug wird der Abdeckung **98** des zweiten Gebindes **92** angenähert.
- Im Containment **4,** rückseitige zweite Zone **42** und Eingangsstation **3,** rückseitige zweite Zone **32:**
   Mit weiterem Schwenken des Auslegers **50** der Apparatur **5** gelangt der Deckel **51** mit der darin integrierten Dekontaminationsvorrichtung **7** auf die zweite Passage **48.** Anschliessend wird mit Abschwenken der zweiten Fördereinrichtung **35** das Behältnis **94** des verarbeiteten ersten Gebindes **91** von der zweiten Passage **48** entfernt und auf die erste Fördereinrichtung **34** zum Abtransport nach ausserhalb der Schleusenanordnung **1** aufgesetzt.

### Ablauf in Prozessphase 8 (Figuren 10A bis 10D)

- In Bereitstellungsstation **2:**
   Wie in Prozessphase 1 gemäss Startposition wird ein nächstes neues, drittes Gebinde **93,** wiederum mit noch verschlossener Umhüllung **99** auf der Auflage **20,** vor dem Zugang **37,** positioniert.
- Im Containment **4,** frontseitige erste Zone **41:**
   Mit dem am Werkzeugkopf **61** des Manipulators **6** installierten Werkzeug wird die Abdeckung **98** des zweiten Gebindes **92,** welches an der ersten Passage **47** angedrückt ist, z.B. durch Aufschneiden geöffnet und der dabei entstehende Schnittabfall entfernt, so dass Zugang zum Durchlass **95** in das Behältnis **94** geschaffen ist.
- In Eingangsstation **3,** rückseitige zweite Zone **32:**
   Ein auf der Bereitstellungsstation **2** von der Umhüllung **99** befreites, weiteres erstes Gebinde **91** mit intakter Abdeckung **98** wurde quer auf die erste Fördereinrichtung **34** transferiert und inzwischen auch auf die zweite Fördereinrichtung **35** geladen.

### Ablauf in Prozessphase 9 (Figuren 11A bis 11D)

- In Bereitstellungsstation **2:**
   Wie in Prozessphase 3 gemäss Figur 5A wird mittels des Werkzeugs **23** die bis anhin verschlossene Umhüllung **99** geöffnet.
- Im Containment **4,** frontseitige erste Zone **41:**
   Wie in Prozessphase 3 gemäss Figur 5B wird mit dem vom Manipulator **6** geführten Werkzeug, bei offener Abdeckung **98,** die optional vorhandene Zwischenlage **97** aus dem zweiten Gebinde **92,** das an der ersten Passage **47** angedrückt ist, entnommen.
- In Eingangsstation **3,** rückseitige zweite Zone **32:**
   Durch Aufschwenken der zweiten Fördereinrichtung **35** wird das weitere erste, quer transferierte Gebinde **91** mit der Abdeckung **98** an die zweite Passage **48** angedrückt.

Somit wird wieder, wie bereits in Prozessphase 3 gemäss Figur 5A, an der ersten Passage **47** entstanden, jetzt an der zweiten Passage **48,** ein zwischen Deckel **51,** Abdeckung **98** des zweiten Gebindes **92,** der die zweite Passage **48** umrandenden Dichtung **480** und möglichen freiliegenden Wandungsflächen ein Umfeld **8** samt davon eingeschlossenes und in der nächsten Prozessphase 10 zu dekontaminierendes Gasvolumen gebildet.

### Ablauf in Prozessphase 10 (Figuren 12A bis 12D)

- In Bereitstellungsstation **2:**
   Hier wiederholt sich die Situation von Prozessphase 4 gemäss Figur 6A, allerdings nun mit dem nächsten neuen, dritten Gebinde **93.**
- Im Containment **4,** frontseitige erste Zone **41:**
   Hier wiederholt sich die Situation von Prozessphase 4 gemäss Figur 6B, allerdings nun mit dem zweiten Gebinde **92,** welches jetzt an der ersten Passage **47** angedrückt ist.
- In Eingangsstation **3,** rückseitige zweite Zone **32,** und Containment **4,** rückseitige zweite Zone **42:**
   Das in der vorherigen Prozessphase 9 gemäss Figur 11B gebildete Umfeld **8** mit darin eingeschlossenem Gasvolumen wird mittels der aktivierten Dekontaminationsvorrichtung **7** dekontaminiert.

### Ablauf in Prozessphase 11 (Figuren 13A bis 13D)

- In Bereitstellungsstation **2:**
   Hier wiederholt sich die Situation von Prozessphase 5 gemäss Figur 7A, allerdings nun mit dem nächsten neuen, dritten Gebinde **93.**
- Im Containment **4,** frontseitige erste Zone **41:**
   Hier wiederholt sich die Situation von Prozessphase 5 gemäss Figur 7B, allerdings wird nun in das Behältnis **94** des zweiten Gebindes **92,** welches jetzt an der ersten Passage **47** angedrückt ist, ein leeres Nest **96** aus der Prozesskammer **43** zurückgeführt.
- In Eingangsstation **3,** rückseitige zweite Zone **32,** und Containment **4,** rückseitige zweite Zone **42:**
   Die Dekontamination läuft weiter.

### Ablauf in Prozessphase 12 (Figuren 14A bis 14D)

- In Bereitstellungsstation **2:**
   Hier wiederholt sich die Situation von Prozessphase 6 gemäss Figur 8A, allerdings nun mit dem nächsten neuen, dritten Gebinde **93.**
- Im Containment **4,** frontseitige erste Zone **41:**
   Hier wiederholt sich die Situation von Prozessphase 6 gemäss Figur 8B, allerdings nun mit dem zweiten Gebinde **92** und an der ersten Passage **47,** der mit Umschwenken des Auslegers **50** der Apparatur **5** der Deckel **51** angenähert wird.
- In Eingangsstation **3,** rückseitige zweite Zone **32,** und Containment **4,** rückseitige zweite Zone **42:**
   Die Dekontamination im Umfeld **8** an der zweiten Passage **48** mit dem weiteren ersten, quer transferierten Gebinde **91** ist beendet. Der Deckel **51** mit der Dekontaminationsvorrichtung **7** sind weggeschwenkt und der Manipulator **6** mit den am Werkzeugkopf **61** installierten Werkzeugen ist an die zweite Passage **48** herangeschwenkt.

### Ablauf in Prozessphase 13 (Figuren 15A bis 15D)

- In Eingangsstation **3,** frontseitige erste Zone **31** und Containment **4,** frontseitige erste Zone **41**:
   Zuerst schwenkt die Apparatur **5** den Deckel **51** auf die erste Passage **47,** diese verschliessend. Anschliessend wird durch Abschwenken der zweiten Fördereinrichtung **35** das Behältnis **94** des verarbeiteten zweiten Gebindes **92** mit dem rückgeführten Nest **96** auf die erste Fördereinrichtung **34** zwecks querverlaufendem Abtransport nach ausserhalb der Schleusenanordnung **1** geladen. Somit wird eine Kollision mit dem nächsten neuen, dritten Gebinde **93** vermieden, welches bereits auf der ersten Fördereinrichtung **34** steht.
- In Eingangsstation **3,** rückseitige zweite Zone **32** und Containment **4,** rückseitige zweite Zone **42:**
   Hier wiederholt sich die Situation wie in Prozessphase 1 gemäss Figur 3B, mit einem Werkzeug am Manipulator **6** wird die Abdeckung **98** geöffnet, allerdings jetzt des ursprünglich quer auf die erste Fördereinrichtung **34** transferierten neuen ersten Gebindes **91.**

### Figuren 16A und 16B

Bei dieser alternativen Anordnung ist die Dekontaminationsvorrichtung **7** nicht, wie bis anhin, im Deckel **51** integriert, sondern das Emissionselement der Dekontaminationsvorrichtung **7,** z.B. eine Vernebelungsdüse zum Eintrag eines H₂O₂-Aerosols, sitzt nicht im Deckel **51,** sondern ausserhalb in einer Halterung **70.** Diese Halterung **70** kann in einfachster Gestalt von einer Abkantung der zwischen Eingangsstation **3** Containment **4** sich erstreckenden Trennwand **40** gebildet sein. Die dem Umfeld **8** zugewandte Oberfläche der Halterung **70** ist dann Bestandteil des im Produktionsprozess zu dekontaminierenden eingeschlossenen Gasvolumens und Umfeld **8.** Unverändert wird der Deckel **51** von einem schwenkbaren Ausleger **50** einer in der Prozesskammer **43** stationierten Apparatur **5** getragen.

## Patentansprüche

1. Verfahren zum Einschleusen von Artikeln **(960)** unter aseptischen Bedingungen aus seriell zugeführten Gebinden **(9;91,92,93)** in eine von einem Gehäuse **(49)** umgebene Prozesskammer **(43)** eines Containments **(4)** mittels einer in einem Aufstellraum **(A)** positionierten Schleusenanordnung **(1),** wobei:
a) der Prozesskammer **(43)** eine Eingangsstation **(3)** vorgelagert ist;
b) ein einzelnes Gebinde **(9;91,92,93)** umfasst:
ba) ein Behältnis **(94)** mit einem aseptischen Innenraum **(940)** und einem Durchlass **(95),** der mit einer Abdeckung **(98)** verschlossen ist; und
bb) die im Innenraum **(940)** gelagerten Artikel **(960),** die nach Öffnen der Abdeckung **(98)** in der Prozesskammer **(43)** zu behandeln sind, **gekennzeichnet durch** die im Produktionsmodus der Schleusenanordnung **(1)** aufeinanderfolgenden Verfahrensschritte:
c) zum Transfer der Artikel **(960)** aus den Gebinden **(9;91,92,93)** in die Prozesskammer **(43) durch** von der Eingangsstation **(3)** zumindest zwei in die Prozesskammer **(43)** führende Passagen **(47,48)** vorgesehen sind und jede der Passagen **(47,48)** wir einerseits von einem in der Prozesskammer **(43)** herangeführten Deckel **(51)** und/oder andererseits von einem in der Eingangsstation **(3)** herangeführten Gebinde **(9;91,92,93)** mit dessen der Passage **(47,48)** zugewandten Abdeckung **(98)** verschlossen;
d) bei an die Passage **(47,48)** herangeführtem Gebinde **(9;91,92,93)** wird die der Passage **(47,48)** zugewandte Oberfläche der Abdeckung **(98)** mit eingeschlossenem Umfeld **(8)** mittels einer Dekontaminationsvorrichtung **(7)** dekontaminiert;
e) nach erfolgter Dekontamination der der Passage **(47,48)** zugewandten Oberfläche der Abdeckung **(98),** einschliesslich deren Umfeld **(8),** wird die Abdeckung **(98)** geöffnet, um die Artikel **(960)** aus dem Behältnis **(94) durch** dessen Durchlass **(95)** in die Prozesskammer **(43)** zu transferieren; und
f) nach Entleerung des Behältnisses **(94)** wird zunächst der Deckel **(51)** wieder an die betreffende Passage **(47,48)** herangebracht, um anschliessend das von den Artikeln **(960)** geleerte Behältnis **(94)** von der Passage **(47,48)** zu entfernen; wobei
g) der Deckel **(51)** an den Passagen **(47,48)** im Wechsel zum Einsatz kommt.

2. Verfahren nach Anspruch 1, **dadurch gekennzeichnet, dass**
a) die Dekontaminationsvorrichtung **(7)** im Deckel **(51)** integriert oder ausserhalb des Deckels **(51)** angeordnet ist; und
b) das Emissionselement der Dekontaminationsvorrichtung **(7)** direkt im Deckel **(51)** oder ausserhalb des Deckels **(51)** in einer Aufnahme **(70)** angeordnet ist.

3. Verfahren nach zumindest einem der Ansprüche 1 und 2, **dadurch gekennzeichnet, dass** das eingeschlossene Umfeld **(8)** das darin vorhandene Gasvolumen und vom Gasvolumen umspülte Oberflächen umfasst, nämlich Oberflächen:
a) des Deckels **(51);**
b) der optionalen Aufnahme **(70);**
c) einer optionalen Dichtung **(470,480);** und
d) von optional freiliegenden Wandungsflächen.

4. Verfahren nach zumindest einem der Ansprüche Anspruch 1 bis 3, **dadurch gekennzeichnet, dass**
a) die Prozesskammer **(43)** mit einem Manipulator **(6)** oder zusätzlich mit einem zweiten Manipulator **(6)** ausgestattet ist, an deren schwenkbaren Auslegern **(60)** jeweils ein mit Werkzeugen bestückter Werkzeugkopf **(61)** sitzt, wobei:
b) die Werkzeuge ausgebildet sind, um die Abdeckung **(98)** zu öffnen und anschliessend die Artikel **(960)** aus dem Behältnis **(94)** durch den freigelegten Durchlass **(95)** in die Prozesskammer **(43)** zu transferieren; und
c) der eine Manipulator **(6)** mit dessen Werkzeugen wechselhaft an den Passagen **(47,48)** zum Öffnen der Abdeckung **(98)** und Transfer der Artikel **(960)** in die Prozesskammer **(43)** zum Einsatz kommt; oder
d) der eine Manipulator **(6)** und der zweite Manipulator **(6)** jeweils nur an einer der Passagen **(47,48)** oder die beiden Manipulatoren **(6)** wechselhaft an den Passagen **(47,48)** zum Einsatz kommen.

5. Verfahren nach zumindest einem der Ansprüche 1 bis 4, **dadurch gekennzeichnet, dass**
a) ein Schneidwerkzeug Komponente der Werkzeuge ist, um für das Öffnen die Abdeckung **(98)** aufzuschneiden; wobei
b) vorzugsweise von der Abdeckung **(98)** und der Umhüllung **(99)** zumindest Flächenanteile aus einem semipermeablen Vliesgewebe, wie Tyvek^{®} bestehen, und die Abdeckung **(98)** vor dem Entfernen den Innenraum **(940)** des Behältnisses **(94)** in sterilem Zustand bewahrt und auf einem den Durchlass **(95)** des Behältnisses **(94)** umlaufenden Rand **(941)** versiegelt ist.

6. Verfahren nach zumindest einem der Ansprüche 1 bis 5, **dadurch gekennzeichnet, dass**
a) im Behältnis **(94)** zur Aufnahme einer Vielzahl von Artikeln **(960)** ein entnehmbares Nest **(96)** lagert, welches zusammen mit den Artikeln **(960)** in die Prozesskammer **(43)** transferiert wird; und
b) nach Leerung des Nestes **(96)** von den Artikeln **(960)** in der Prozesskammer **(43),** dieses geleerte Nest **(96)** aus der Prozesskammer **(43)** in ein nächstes oder dasselbe an der betreffenden Passage **(47,48)** angedrücktes geleertes Behältnis **(94)** entsorgt wird; oder
c) die Artikel **(960)** direkt aus dem im Behältnis **(94)** verbleibenden Nest **(96)** in die Prozesskammer **(43)** transferiert werden.

7. Verfahren nach zumindest einem der Ansprüche 1 bis 6, **dadurch gekennzeichnet, dass** die Dekontaminationsvorrichtung vorzugsweise als Strahlungsquelle, z.B. UVC, UV, E-Beam oder Puls-Licht, oder alternativ als eine Begasungseinrichtung, z.B zur Verneblung einer H₂O₂-Lösung, ausgebildet wird.

8. Verfahren nach zumindest einem der Ansprüche 1 bis 7, **dadurch gekennzeichnet, dass** der Eingangsstation **(3),** welche von einem Gehäuse **(39)** umgeben ist, das einen zu einer angrenzenden Bereitstellungsstation **(2)** führenden Zugang **(37)** aufweist, wobei:
a) die Eingangsstation **(3)** mit einer gereinigten, gleichgerichteten Verdrängungsströmung **(LF)** betrieben wird, die teilweise durch den Zugang **(37)** austritt;
b) an der Bereitstellungsstation **(2)** die Gebinde **(9;91,92,93)** angeliefert werden, welche im Anlieferungszustand jeweils von einer Umhüllung **(99)** umgeben sind, um das Innenvolumen **(90)** des Gebindes **(9;91,92,93)** mit dessen Inhalt aseptisch zu erhalten; und
c) an der Bereitstellungsstation **(2)** eine an der Umhüllung **(99)** angebrachte Öffnung, die man bis anhin geschlossen hält, nun auf der der aus dem Zugang **(37)** austretenden Verdrängungsströmung **(LF)** zugewandten Seite freigegeben wird, um das einzelne Gebinde **(9;91,92,93)** in die Eingangsstation **(3)** einzubringen; oder
d) an der Bereitstellungsstation **(2)** die Umhüllung **(99)** auf der der aus dem Zugang **(37)** austretenden Verdrängungsströmung **(LF)** zugewandten Seite aufgetrennt wird, um das einzelne Gebinde **(9;91,92,93)** in die Eingangsstation **(3)** einzubringen.

9. Verfahren nach Anspruch 8, **dadurch gekennzeichnet, dass**
a) die Positionierung des in der zuerst noch geschlossenen Umhüllung **(99)** steckenden Gebindes **(9;91,92,93)** auf einer Auflage **(20)** erfolgt;
b) zum Ausschieben des Gebindes **(9;91,92,93)** aus der offenen Umhüllung **(99),** bei auf das Gebinde **(9;91,92,93)** fliessender Verdrängungsströmung **(LF),** ein Vorschubmittel **(22)** dient; und
c) die Umhüllung **(99)** von einem Greifer **(21),** vorzugsweise als Sauger ausgebildet, gehalten wird.

10. Verfahren nach zumindest einem der Ansprüche 1 bis 9, **dadurch gekennzeichnet, dass**
a) die in die Eingangsstation **(3)** eingebrachten Gebinde **(9;91,92,93)** mittels zumindest einer Fördereinrichtung **(34,35)** den Passagen **(47,48)** zugeführt werden; und
b) die zumindest eine Fördereinrichtung **(34,35)** vorzugsweise auf dem Prinzip der Magnetschwebetechnik beruht.

11. Verfahren nach Anspruch 10, **dadurch gekennzeichnet, dass**
a) mittels einer ersten Fördereinrichtung **(34)** der Transport der Gebinde **(9;91,92,93)** innerhalb der Eingangsstation **(3)** erfolgt; während
b) eine zweite Fördereinrichtung **(35)** das Heranschwenken der Gebinde **(9;91,92,93)** mit an der jeweiligen Passage **(47,48)** angepressten Abdeckung **(98)** bewirkt; und
c) die zweite Fördereinrichtung **(35)** auch zum Entfernen bearbeiteter Gebinde **(9;91,92,93)** von der jeweiligen Passage **(47,48)** nutzbar ist.

12. Verfahren nach Anspruch 11, **dadurch gekennzeichnet, dass** zur Positionierung der Gebinde **(9;91,92,93)** an der jeweiligen Passage **(47,48)** die zweite Fördereinrichtung **(35)** ein kardanisches Lager **(351)** umfasst.

13. Verfahren nach zumindest einem der Ansprüche 11 und 12, **dadurch gekennzeichnet, dass** die zweite Fördereinrichtung **(35)** besteht aus:
a) einer Achse **(350),** durch welche sich eine erste Drehachse **(D1)** erstreckt;
b) zwei nebeneinander an der Achse **(350)** fixierten Gabeln **(352),** die jeweils um eine zweite Drehachse **(D2)** schwenkbar sind, welche sich rechtwinklig zur ersten Drehachse **(D1)** erstrecken; und
c) jeweils einen in der zugehörigen Gabel **(352)** aufgehängten und um eine dritte Drehachse **(D3)** schwenkbaren Träger **(353),** wobei die erste Drehachse **(D1)** und die dritte Drehachse **(D3)** zueinander parallel verlaufen und die Träger **(353)** zum Unterfassen der Ränder **(941)** der Behältnisse **(94)** beim An- bzw. Abschwenken von den Passagen **(47,48)** konfiguriert sind; wobei
d) zwei nebeneinander liegende kardanische Lager **(351)** jeweils gebildet sind aus einen U-förmigen Träger **(353),** der in der dritten Drehachse **(D3)** jeweils mit einer Gabel **(352)** verbunden ist, die in der zweiten Drehachse **(D2)** aufgehängt ist.

14. Verfahren nach zumindest einem der Ansprüche 1 bis 13, **dadurch gekennzeichnet, dass** in jeder Passage **(47,48)** eine Dichtung **(470,480)** sitzt, welche zusammen mit der Abdeckung **(98)** eines temporär angepressten Gebindes **(9;91,92,93)** und/oder mit einem temporär angepressten Deckel **(51)** an den Passagen **(47,48),** im Übergang von der Eingangsstation **(3)** zur Prozesskammer **(43),** Abdichtungen schafft.

15. Verfahren nach zumindest einem der Ansprüche 1 bis 14, **dadurch gekennzeichnet, dass** der Deckel **(51)** von einem schwenkbaren Ausleger **(50)** einer in der Prozesskammer **(43)** positionierten Apparatur **(5)** getragen wird.

16. Verfahren nach zumindest einem der Ansprüche 4 bis 15, **dadurch gekennzeichnet, dass** der zumindest eine Manipulator **(6)** oder auch der zusätzliche Manipulator **(6)** als Roboter ausgebildet sind.

17. Verfahren nach zumindest einem der Ansprüche 1 bis 16, **dadurch gekennzeichnet, dass** die Behältnisse **(94)** der Gebinde **(9;91,92,93)** die Gestalt wannenförmiger Tubs haben und die Artikel **(960),** z.B. Phiolen, Vials oder Spritzen sind, die in systematisch angeordneten Aufnahmekonturen von Nestern **(96)** stecken.

18. Schleusenanordnung zum Einschleusen von Artikeln **(960)** unter aseptischen Bedingungen aus seriell zugeführten Gebinden **(9;91,92,93)** in eine von einem Gehäuse **(49)** umgebene Prozesskammer **(43)** eines Containments **(4)** mittels einer in einem Aufstellraum **(A)** positionierten Schleusenanordnung **(1),** wobei:
a) der Prozesskammer **(43)** eine Eingangsstation **(3)** vorgelagert ist;
b) ein einzelnes Gebinde **(9;91,92,93)** umfasst:
ba) ein Behältnis **(94)** mit einem aseptischen Innenraum **(940)** und einem Durchlass **(95),** der mit einer Abdeckung **(98)** verschlossen ist; und
bb) die im Innenraum **(940)** gelagerten Artikel **(960),** die nach Öffnen der Abdeckung **(98)** in der Prozesskammer **(43)** zu behandeln sind, **dadurch gekennzeichnet, dass**:
c) zum Transfer der Artikel **(960)** aus den Gebinden **(9;91,92,93)** in die Prozesskammer **(43)** durch von der Eingangsstation **(3)** zumindest zwei in die Prozesskammer **(43)** führende Passagen **(47,48)** vorgesehen sind und jede der Passagen **(47,48)** einerseits von einem in der Prozesskammer **(43)** herangeführten Deckel **(51)** und/oder andererseits von einem in der Eingangsstation **(3)** herangeführten Gebinde **(9;91,92,93)** mit dessen der Passage **(47,48)** zugewandten Abdeckung **(98)** verschliessbar ist;
d) bei an die Passage **(47,48)** herangeführtem Gebinde (**9**;**91**,**92**,**93**) die der Passage **(47,48)** zugewandte Oberfläche der Abdeckung **(98)** mit eingeschlossenem Umfeld **(8)** mittels einer Dekontaminationsvorrichtung **(7)** dekontaminierbar ist;
e) nach erfolgter Dekontamination der der Passage **(47,48)** zugewandten Oberfläche der Abdeckung **(98),** einschliesslich deren Umfeld **(8),** sich die Abdeckung **(98)** öffnen lässt und die Artikel **(960)** aus dem Behältnis **(94)** durch dessen Durchlass **(95)** in die Prozesskammer **(43)** transferierbar sind; und
f) der Deckel **(51)** wieder an die betreffende Passage **(47,48),** an welcher das geleerte Behältnis **(94)** ansteht, anfügbar ist und sich somit das leere Behältnis **(94)** von der Passage **(47,48)** entfernen lässt; wobei
g) der Deckel **(51)** im Wechsel zwischen den Passagen **(47,48)** zum Einsatz kommt.

19. Schleusenanordnung nach Anspruch 18, **dadurch gekennzeichnet, dass**
a) die Dekontaminationsvorrichtung **(7)** im Deckel **(51)** integriert oder ausserhalb des Deckels **(51)** angeordnet ist; und
b) das Emissionselement der Dekontaminationsvorrichtung **(7)** direkt im Deckel **(51)** oder ausserhalb des Deckels **(51)** in einer Aufnahme **(70)** angeordnet ist.

20. Schleusenanordnung nach zumindest einem der Ansprüche 18 und 19, **dadurch gekennzeichnet, dass** das eingeschlossene Umfeld **(8)** das darin vorhandene Gasvolumen und vom Gasvolumen umspülte Oberflächen umfasst, nämlich Oberflächen:
a) des Deckels **(51);**
b) der optionalen Aufnahme **(70);**
c) einer optionalen Dichtung **(470,480);** und
d) von optional freiliegenden Wandungsflächen.

21. Schleusenanordnung nach zumindest einem der Ansprüche Anspruch 18 bis 20, **dadurch gekennzeichnet, dass**
a) die Prozesskammer **(43)** mit einem Manipulator **(6)** oder zusätzlich mit einem zweiten Manipulator **(6)** ausgestattet ist, an deren schwenkbaren Auslegern **(60)** jeweils ein mit Werkzeugen bestückter Werkzeugkopf **(61)** sitzt, wobei:
b) die Werkzeuge ausgebildet sind, um die Abdeckung **(98)** zu öffnen und anschliessend die Artikel **(960)** aus dem Behältnis **(94)** durch den freigelegten Durchlass **(95)** in die Prozesskammer **(43)** zu transferieren; und
c) der eine Manipulator **(6)** mit dessen Werkzeugen wechselhaft an den Passagen **(47,48)** zum Öffnen der Abdeckung **(98)** und Transfer der Artikel **(960)** in die Prozesskammer **(43)** zum Einsatz kommt; oder
d) der eine Manipulator **(6)** und der zweite Manipulator **(6)** jeweils nur an einer der Passagen **(47,48)** oder die beiden Manipulatoren **(6)** wechselhaft an den Passagen **(47,48)** zum Einsatz kommen.

22. Schleusenanordnung nach zumindest einem der Ansprüche 18 bis 21, **dadurch gekennzeichnet, dass**
a) die Werkzeuge ein Schneidwerkzeug als Komponente aufweisen, bestimmt zum Öffnen der Abdeckung **(98);** wobei
b) vorzugsweise von der Abdeckung **(98)** und der Umhüllung **(99)** zumindest Flächenanteile aus einem semipermeablen Vliesgewebe, wie Tyvek^{®} bestehen, und die Abdeckung **(98)** vor dem Entfernen den Innenraum **(940)** des Behältnisses **(94)** in sterilem Zustand bewahrt und auf einem den Durchlass **(95)** des Behältnisses **(94)** umlaufenden Rand **(941)** versiegelt ist.

23. Schleusenanordnung nach zumindest einem der Ansprüche 18 bis 22, **dadurch gekennzeichnet, dass**
a) im Behältnis **(94)** zur Aufnahme einer Vielzahl von Artikeln **(960)** ein entnehmbares Nest **(96)** lagert, welches zusammen mit den Artikeln **(960)** in die Prozesskammer **(43)** transferierbar ist; und
b) das von Artikeln **(960)** geleerte Nest **(96)** aus der Prozesskammer **(43)** in ein nächstes oder dasselbe an der betreffenden Passage **(47,48)** angedocktes geleertes Behältnis **(94)** entsorgbar ist; oder
c) die Artikel **(960)** direkt aus dem im Behältnis **(94)** verbleibenden Nest **(96)** in die Prozesskammer **(43)** transferierbar sind.

24. Schleusenanordnung nach zumindest einem der Ansprüche 18 bis 23, **dadurch gekennzeichnet, dass** die Dekontaminationsvorrichtung vorzugsweise als Strahlungsquelle, z.B. UVC, UV, E-Beam oder Puls-Licht, oder alternativ als eine Begasungseinrichtung, z.B zur Verneblung einer H₂O₂-Lösung, ausgebildet ist.

25. Schleusenanordnung nach zumindest einem der Ansprüche 18 bis 24, **dadurch gekennzeichnet, dass** der Eingangsstation **(3),** welche von einem Gehäuse **(39)** umgeben ist, das einen zu einer angrenzenden Bereitstellungsstation **(2)** führenden Zugang **(37)** aufweist, wobei:
a) die Eingangsstation **(3)** mit einer gereinigten, gleichgerichteten Verdrängungsströmung **(LF)** betreibbar ist, die teilweise durch den Zugang **(37)** austritt;
b) die Bereitstellungsstation **(2)** zur Anlieferung der Gebinde **(9;91,92,93)** bestimmt ist, welche im Anlieferungszustand jeweils von einer Umhüllung **(99)** umgeben sind, um das Innenvolumen **(90)** des Gebindes **(9;91,92,93)** mit dessen Inhalt aseptisch zu erhalten; und
c) an der Bereitstellungsstation **(2)** eine an der Umhüllung **(99)** angebrachte Öffnung, die bis anhin mittels eines angesetzten Werkzeugs geschlossen bleibt, nun auf der der aus dem Zugang **(37)** austretenden Verdrängungsströmung **(LF)** zugewandten Seite durch Lösen des Werkzeugs freikommt, so dass das einzelne Gebinde **(9;91,92,93)** in die Eingangsstation **(3)** einbringbar ist; oder
d) an der Bereitstellungsstation **(2)** die Umhüllung **(99)** auf der der aus dem Zugang **(37)** austretenden Verdrängungsströmung **(LF)** zugewandten Seite mittels eines Werkzeugs auftrennbar ist, so dass das einzelne Gebinde **(9;91,92,93)** in die Eingangsstation **(3)** einbringbar ist.

26. Schleusenanordnung nach Anspruch 25, **dadurch gekennzeichnet, dass**
a) zur Positionierung des in der zuerst noch geschlossenen Umhüllung **(99)** steckenden Gebindes (**9**;**91**,**92**,**93**) eine Auflage **(20)** vorgesehen ist;
b) zum Ausschieben des Gebindes (**9**;**91**,**92**,**93**) aus der offenen Umhüllung **(99),** bei auf das Gebinde (**9**;**91**,**92**,**93**) fliessender Verdrängungsströmung **(LF),** ein Vorschubmittel **(22)** dient; und
c) zum Zurückhalten der Umhüllung **(99)** ein Greifer **(21),** vorzugsweise als Sauger ausgebildet, dient.

27. Schleusenanordnung nach zumindest einem der Ansprüche 18 bis 26, **dadurch gekennzeichnet, dass**
a) zumindest eine Fördereinrichtung **(34,35)** zur Zuführung der in die Eingangsstation **(3)** eingebrachten Gebinde (**9**;**91**,**92**,**93**) an die Passagen **(47,48)** vorgesehen ist; und
b) die zumindest eine Fördereinrichtung **(34,35)** auf dem Prinzip der Magnetschwebetechnik beruht.

28. Schleusenanordnung nach Anspruch 27, **dadurch gekennzeichnet, dass**
a) zum Transport der Gebinde (**9**;**91**,**92**,**93**) innerhalb der Eingangsstation **(3)** eine erste Fördereinrichtung **(34)** dient; während
b) eine zweite Fördereinrichtung **(35)** zum Heranschwenken der Gebinde **(9;91,92,93)** mit an der jeweiligen Passage **(47,48)** angepressten Abdeckung **(98)** bestimmt ist; und
c) die zweite Fördereinrichtung **(35)** auch zum Entfernen bearbeiteter Gebinde **(9;91,92,93)** von der jeweiligen Passage **(47,48)** nutzbar ist.

29. Schleusenanordnung nach Anspruch 28, **dadurch gekennzeichnet, dass** zur Positionierung der Gebinde (**9**;**91**,**92**,**93**) an der jeweiligen Passage **(47,48)** die zweite Fördereinrichtung **(35)** ein kardanisches Lager **(351)** umfasst.

30. Schleusenanordnung nach zumindest einem der Ansprüche 28 und 29, **dadurch gekennzeichnet, dass** die zweite Fördereinrichtung **(35)** besteht aus:
a) einer Achse **(350),** durch welche sich eine erste Drehachse **(D1)** erstreckt;
b) zwei nebeneinander an der Achse **(350)** fixierten Gabeln **(352),** die jeweils um eine zweite Drehachse **(D2)** schwenkbar sind, welche sich rechtwinklig zur ersten Drehachse **(D1)** erstrecken; und
c) jeweils einen in der zugehörigen Gabel **(352)** aufgehängten und um eine dritte Drehachse **(D3)** schwenkbaren Träger **(353),** wobei die erste Drehachse **(D1)** und die dritte Drehachse **(D3)** zueinander parallel verlaufen und die Träger **(353)** zum Unterfassen der Ränder **(941)** der Behältnisse **(94)** beim An- bzw. Abschwenken von den Passagen **(47,48)** konfiguriert sind; wobei
d) zwei nebeneinander liegende kardanische Lager **(351)** jeweils gebildet sind aus einen U-förmigen Träger **(353),** der in der dritten Drehachse **(D3)** jeweils mit einer Gabel **(352)** verbunden ist, die in der zweiten Drehachse **(D2)** aufgehängt ist.

31. Schleusenanordnung nach zumindest einem der Ansprüche 18 bis 30, **dadurch gekennzeichnet, dass** in jeder Passage **(47,48)** eine Dichtung **(470,480)** sitzt, welche zusammen mit der Abdeckung **(98)** eines temporär angepressten Gebindes **(9;91,92,93)** und/oder mit einem temporär angepressten Deckel **(51)** an den Passagen **(47,48),** im Übergang von der Eingangsstation **(3)** zur Prozesskammer **(43),** Abdichtungen schafft.

32. Schleusenanordnung nach zumindest einem der Ansprüche 18 bis 31, **dadurch gekennzeichnet, dass** ein schwenkbarer Ausleger **(50)** einer in der Prozesskammer **(43)** positionierten Apparatur **(5)** den Deckel **(51)** trägt.

33. Schleusenanordnung nach zumindest einem der Ansprüche 21 bis 32, **dadurch gekennzeichnet, dass** der zumindest eine Manipulator **(6)** oder auch der zusätzliche Manipulator **(6)** als Roboter ausgebildet sind.

34. Schleusenanordnung nach zumindest einem der Ansprüche 18 bis 33, **dadurch gekennzeichnet, dass** die Behältnisse **(94)** der Gebinde (**9**;**91**,**92**,**93**) die Gestalt wannenförmiger Tubs haben und die Artikel **(960),** z.B. Phiolen, Vials oder Spritzen sind, die in systematisch angeordneten Aufnahmekonturen von Nestern **(96)** stecken.

## Claims

1. Method for feeding articles **(960)** under aseptic conditions from serially supplied containers **(9; 91, 92, 93)** into a process chamber **(43)** of a containment **(4)** surrounded by a housing **(49)** by means of a sluice arrangement **(1)** positioned in an installation space **(A),** wherein:
a) an input station **(3)** is positioned upstream of the process chamber **(43);**
b) a single container **(9; 91, 92, 93)** comprises:
ba) a receptacle **(94)** having an aseptic interior **(940)** and a passage **(95)** closed by a cover **(98);** and
bb) the articles **(960)** stored in the interior **(940),** which are to be treated in the process chamber **(43)** after opening the cover **(98),** **characterized by** the successive method steps in the production mode of the sluice arrangement (**1**):
c) at least two passages **(47, 48)** leading from the input station **(3)** into the process chamber **(43)** are provided for transferring the articles **(960)** from the containers **(9; 91, 92, 93)** into the process chamber **(43),** and each of the passages **(47, 48)** is closed on the one hand by a lid **(51)** brought up in the process chamber **(43)** and/or on the other hand by a container **(9; 91, 92, 93)** brought up in the input station **(3)** with its cover **(98)** facing the passage **(47, 48);**
d) when the container **(9; 91, 92, 93)** is brought up to the passage **(47, 48),** the surface of the cover **(98)** facing the passage **(47, 48)** with enclosed environment **(8)** is decontaminated by means of a decontamination device **(7);**
e) after decontamination of the surface of the cover **(98)** facing the passage **(47, 48),** including its environment **(8),** the cover **(98)** is opened to transfer the articles **(960)** from the receptacle **(94)** through its passage **(95)** into the process chamber **(43);** and
f) after the receptacle **(94)** has been emptied, the lid **(51)** is first brought back to the passage **(47, 48)** in question in order to subsequently remove the receptacle **(94)** emptied of the articles **(960)** from the passage **(47, 48);** wherein
g) the lid **(51)** is used alternately at the passages **(47, 48**).

2. Method according to claim 1, **characterized in that**
a) the decontamination device **(7)** is integrated in the lid **(51)** or is arranged outside of the lid **(51);** and
b) the emission element of the decontamination device **(7)** is arranged directly in the lid **(51)** or outside the lid **(51)** in a holder **(70).**

3. Method according to at least one of claims 1 and 2, **characterized in that** the enclosed environment **(8)** comprises the gas volume present therein and surfaces surrounded by the gas volume, namely surfaces:
a) of the lid **(51);**
b) of the optional holder **(70);**
c) of an optional seal **(470,480);** and
d) of optionally exposed wall surfaces.

4. Method according to at least one of claims 1 to 3, **characterized in that**
a) the process chamber **(43)** is equipped with a manipulator **(6)** or additionally with a second manipulator **(6),** on each of whose pivotable cantilevers **(60)** a tool head **(61)** fitted with tools is mounted, wherein:
b) the tools are adapted to open the cover **(98)** and subsequently transfer the articles **(960)** from the receptacle **(94)** through the exposed passage **(95)** into the process chamber **(43);** and
c) the one manipulator **(6)** with its tools is used alternately at the passages **(47, 48)** for opening the cover **(98)** and transferring the articles **(960)** into the process chamber **(43);** or
d) the one manipulator **(6)** and the second manipulator **(6)** are each used at only one of the passages **(47, 48)** or the two manipulators **(6)** are used alternately at the passages **(47, 48**).

5. Method according to at least one of claims 1 to 4, **characterized in that**
a) a cutting tool is a component of the tools to cut the cover **(98)** for opening; wherein
b) preferably at least surface portions of the cover **(98)** and the enclosure **(99)** consist of a semipermeable nonwoven fabric, such as Tyvek^{®}, and the cover **(98),** before removal, preserves the interior **(940)** of the receptacle **(94)** in a sterile state and is sealed on an edge **(941)** surrounding the passage **(95)** of the receptacle (**94**).

6. Method according to at least one of claims 1 to 5, **characterized in that**
a) a removable nest **(96)** is stored in the receptacle **(94)** for accommodating a plurality of articles **(960)** and is transferred to the process chamber **(43)** together with the articles **(960);** and
b) after emptying the nest **(96)** of the articles **(960)** in the process chamber **(43),** this emptied nest **(96)** is disposed of from the process chamber **(43)** into a next or the same emptied receptacle **(94)** pressed against the respective passage **(47, 48);** or
c) the articles **(960)** are transferred directly from the nest **(96)** remaining in the receptacle **(94)** into the process chamber **(43).**

7. Method according to at least one of claims 1 to 6, **characterized in that** the decontamination device is preferably designed as a radiation source, e.g. UVC, UV, e-beam or pulsed light, or alternatively as a fumigation device, e.g. for nebulizing an H₂O₂ solution.

8. Method according to at least one of claims 1 to 7, **characterized in that** the input station **(3),** which is surrounded by a housing **(39)** having an access **(37)** leading to an adjacent supply station **(2),** wherein:
a) the inlet station **(3)** is operated with a purified, rectified displacement flow **(LF),** which partially exits through the access **(37);**
b) the containers **(9; 91, 92, 93)** are delivered to the supply station **(2),** which in the delivery state are each surrounded by an enclosure **(99)** in order to aseptically maintain the internal volume **(90)** of the container **(9; 91, 92, 93)** with its contents; and
c) at the supply station **(2),** an opening provided on the enclosure **(99),** which was previously kept closed, is now opened on the side facing the displacement flow **(LF)** emerging from the access **(37)** in order to introduce the individual container **(9; 91, 92, 93)** into the input station **(3);** or
d) at the supply station **(2),** the enclosure **(99)** is separated on the side facing the displacement flow **(LF)** emerging from the access **(37)** in order to introduce the individual container **(9; 91, 92, 93)** into the input station **(3).**

9. Method according to claim 8, **characterized in that**
a) the container **(9; 91, 92, 93),** which is still in the initially closed enclosure **(99),** is positioned on a support **(20);**
b) an advancing means **(22)** is used for pushing the container **(9; 91, 92, 93)** out of the open enclosure **(99)** with a displacement flow **(LF)** flowing onto the container **(9; 91, 92, 93);** and
c) the enclosure **(99)** is retained by a gripper **(21),** preferably designed as a suction cup.

10. Method according to at least one of claims 1 to 9, **characterized in that**
a) the containers **(9; 91, 92, 93)** introduced into the input station **(3)** are fed to the passages **(47, 48)** by means of at least one conveying device **(34, 35);** and
b) the at least one conveying device **(34, 35)** is preferably based on the principle of magnetic levitation.

11. Method according to claim 10, **characterized in that**
a) the transport of the containers **(9; 91, 92, 93)** within the input station **(3)** takes place by means of a first conveying device **(34);** while
b) a second conveying device **(35)** causes the containers **(9; 91, 92, 93)** with the cover **(98)** pressed against the respective passage **(47, 48)** to be swung towards it; and
c) the second conveying device **(35)** can also be used to remove processed containers **(9; 91, 92, 93)** from the respective passage **(47, 48).**

12. Method according to claim 11, **characterized in that** the second conveying device **(35)** comprises a cardanic bearing **(351)** for positioning the containers **(9; 91, 92, 93)** at the respective passage **(47, 48).**

13. Method according to at least one of claims 11 and 12, **characterized in that** the second conveying device **(35)** consists of:
a) an axle **(350)** through which a first axis of rotation **(D1)** extends;
b) two forks **(352)** fixed side by side on the axle **(350),** each pivotable about a second axis of rotation **(D2)** extending at right angles to the first axis of rotation **(D1);** and
c) in each case a carrier **(353)** suspended in the assigned fork **(352)** and pivotable about a third axis of rotation **(D3),** wherein the first axis of rotation **(D1)** and the third axis of rotation **(D3)** extend parallel to one another and the carriers **(353)** are configured for engaging under the edges **(941)** of the receptacles **(94)** when pivoting to and away from the passages **(47, 48);** wherein
d) two adjacent cardanic bearings **(351)** are each formed from a U-shaped carrier **(353)** which is connected in the third axis of rotation **(D3)** in each case to a fork **(352)** which is suspended in the second axis of rotation **(D2).**

14. Method according to at least one of claims 1 to 13, **characterized in that** in each passage **(47, 48)** there is a seal **(470, 480)** which, together with the cover **(98)** of a temporarily pressed-on container **(9; 91, 92, 93)** and/or with a temporarily pressed-on lid **(51),** creates seals at the passages **(47, 48)** in the transition from the input station **(3)** to the process chamber **(43).**

15. Method according to at least one of claims 1 to 14, **characterized in that** the lid **(51)** is carried by a pivotable cantilever **(50)** of an apparatus **(5)** positioned in the process chamber **(43).**

16. Method according to at least one of claims 4 to 15, **characterized in that** the at least one manipulator **(6)** or also the additional manipulator **(6)** is designed as a robot.

17. Method according to at least one of claims 1 to 16, **characterized in that** the receptacles **(94)** of the containers **(9; 91, 92, 93)** have the shape of trough-shaped tubs and the articles **(960),** for example vials or syringes, are inserted in systematically arranged receiving contours of nests (**96**).

18. Sluice arrangement for transferring articles **(960)** under aseptic conditions from serially supplied containers **(9; 91, 92, 93)** into a process chamber **(43)** of a containment **(4)** surrounded by a housing **(49)** by means of a sluice arrangement **(1)** positioned in an installation space **(A),** wherein:
a) an input station **(3)** is located upstream of the process chamber **(43);**
b) a single container (**9**;**91**,**92**,**93**) comprises:
ba) a receptacle **(94)** having an aseptic interior **(940)** and a passage **(95)** which is closed with a cover **(98);** and
bb) the articles **(960)** stored in the interior **(940),** which are to be treated in the process chamber **(43)** after opening the cover **(98),** **characterized in that**:
c) at least two passages **(47, 48)** leading from the input station **(3)** into the process chamber **(43)** are provided for transferring the articles **(960)** from the containers **(9; 91, 92, 93)** into the process chamber **(43),** and each of the passages **(47, 48)** can be closed on the one hand by a lid **(51)** brought up in the process chamber **(43)** and/or on the other hand by a container **(9; 91, 92, 93)** brought up in the input station **(3)** with its cover **(98)** facing the passage **(47, 48);**
d) when the container **(9; 91, 92, 93)** is brought up to the passage **(47, 48),** the surface of the cover **(98)** facing the passage **(47, 48)** with enclosed environment **(8)** can be decontaminated by means of a decontamination device **(7);**
e) after decontamination of the surface of the cover **(98)** facing the passage **(47, 48),** including its environment **(8),** the cover **(98)** can be opened and the articles **(960)** can be transferred from the receptacle **(94)** through its passage **(95)** into the process chamber **(43);** and
f) the lid **(51)** can be reattached to the respective passage **(47, 48)** at which the emptied receptacle **(94)** abuts, and thus the empty receptacle **(94)** can be removed from the passage **(47, 48);** wherein
g) the lid **(51)** is used alternately between the passages (**47**, **48**).

19. Sluice arrangement according to claim 18, **characterized in that**
a) the decontamination device **(7)** is integrated in the lid **(51)** or is arranged outside the lid **(51);** and
b) the emission element of the decontamination device **(7)** is arranged directly in the lid **(51)** or outside the lid **(51)** in a holder (**70**).

20. Sluice arrangement according to at least one of claims 18 and 19, **characterized in that** the enclosed environment **(8)** comprises the gas volume present therein and surfaces surrounded by the gas volume, namely surfaces:
a) of the lid **(51);**
b) of the optional holder **(70);**
c) an optional seal **(470,480);** and
d) of optionally exposed wall surfaces.

21. Sluice arrangement according to at least one of claims 18 to 20, **characterized in that**
a) the process chamber **(43)** is equipped with a manipulator **(6)** or additionally with a second manipulator **(6),** on each of whose pivotable cantilevers **(60)** a tool head **(61)** fitted with tools is mounted, wherein:
b) the tools are adapted to open the cover **(98)** and subsequently transfer the articles **(960)** from the receptacle **(94)** through the exposed passage **(95)** into the process chamber **(43);** and
c) the one manipulator **(6)** with its tools is used alternately at the passages **(47, 48)** for opening the cover **(98)** and transferring the articles **(960)** into the process chamber **(43);** or
d) the one manipulator **(6)** and the second manipulator **(6)** are each used at only one of the passages **(47, 48)** or the two manipulators **(6)** are used alternately at the passages **(47, 48).**

22. Sluice arrangement according to at least one of claims 18 to 21, **characterized in that**
a) the tools have a cutting tool as a component intended for opening the cover **(98);** wherein
b) preferably at least surface portions of the cover **(98)** and the enclosure **(99)** consist of a semipermeable nonwoven fabric, such as Tyvek^{®}, and the cover **(98),** before removal, preserves the interior **(940)** of the receptacle **(94)** in a sterile state and is sealed on an edge **(941)** surrounding the passage **(95)** of the receptacle (**94**).

23. Sluice arrangement according to at least one of claims 18 to 22, **characterized in that**
a) the receptacle **(94)** for accommodating a plurality of articles **(960)** stores a removable nest **(96)** which is transferable to the process chamber **(43)** together with the articles **(960);** and
b) the nest **(96)** emptied of articles **(960)** is disposable from the process chamber **(43)** into a next or the same emptied receptacle **(94)** docked at the respective passage **(47, 48);** or
c) the articles **(960)** can be transferred directly from the nest **(96)** remaining in the receptacle **(94)** into the process chamber **(43).**

24. Sluice arrangement according to at least one of claims 18 to 23, **characterized in that** the decontamination device is preferably designed as a radiation source, e.g. UVC, UV, e-beam or pulsed light, or alternatively as a fumigation device, e.g. for nebulizing an H₂O₂ solution.

25. Sluice arrangement according to at least one of claims 18 to 24, **characterized in that** the input station **(3),** which is surrounded by a housing **(39)** having an access **(37)** leading to an adjacent supply station **(2),** wherein:
a) the inlet station **(3)** can be operated with a purified, rectified displacement flow **(LF),** which partially exits through the access **(37);**
b) the supply station **(2)** is intended for the delivery of the containers **(9; 91, 92, 93),** which in the delivery state are each surrounded by an enclosure **(99)** in order to aseptically maintain the internal volume **(90)** of the container **(9; 91, 92, 93)** with its contents; and
c) at the supply station **(2),** an opening provided on the enclosure **(99),** which was previously kept closed by means of an attached tool, is now opened on the side facing the displacement flow **(LF)** emerging from the access **(37)** by releasing the tool, so that the individual container **(9; 91, 92, 93)** can be introduced into the input station **(3);** or
d) at the supply station **(2),** the enclosure **(99)** can be separated by means of a tool on the side facing the displacement flow **(LF)** emerging from the access **(37),** so that the individual container **(9; 91, 92, 93)** can be introduced into the input station **(3).**

26. Sluice arrangement according to claim **25, characterized in that**
a) a support **(20)** is provided for positioning the container **(9; 91, 92, 93)** which is inserted in the initially still closed enclosure **(99);**
b) an advancing means **(22)** is used for pushing the container **(9; 91, 92, 93)** out of the open enclosure **(99)** with a displacement flow **(LF)** flowing onto the container **(9; 91, 92, 93);** and
c) a gripper **(21),** preferably designed as a suction cup, is used to retain the enclosure **(99).**

27. Sluice arrangement according to at least one of claims 18 to 26, **characterized in that**
a) at least one conveying device **(34, 35)** is provided for feeding the containers **(9; 91, 92, 93)** introduced into the input station **(3)** to the passages **(47, 48);** and
b) the at least one conveying device **(34 35)** is based on the principle of magnetic levitation.

28. Sluice arrangement according to claim 27, **characterized in that**
a) a first conveying device **(34)** is used to transport the containers **(9; 91, 92, 93)** within the input station **(3);** while
b) a second conveying device **(35)** is intended for pivoting the containers **(9; 91, 92, 93)** with the cover **(98)** pressed against the respective passage **(47, 48);** and
c) the second conveying device **(35)** can also be used to remove processed containers **(9; 91, 92, 93)** from the respective passage (**47**, **48**).

29. Sluice arrangement according to claim 28, **characterized in that** the second conveying device **(35)** comprises a cardanic bearing **(351)** for positioning the containers **(9; 91, 92, 93)** at the respective passage **(47, 48).**

30. Sluice arrangement according to at least one of claims 28 and 29, **characterized in that** the second conveying device **(35)** consists of:
a) an axle **(350)** through which a first axis of rotation **(D1)** extends;
b) two forks **(352)** fixed side by side on the axle **(350),** each pivotable about a second axis of rotation **(D2)** extending at right angles to the first axis of rotation **(01);** and
c) in each case a carrier **(353)** suspended in the assigned fork **(352)** and pivotable about a third axis of rotation **(D3),** wherein the first axis of rotation **(D1)** and the third axis of rotation **(D3)** extend parallel to one another and the carriers **(353)** are configured for engaging under the edges **(941)** of the receptacles **(94)** when pivoting to and away from the passages **(47, 48);** wherein
d) two adjacent cardanic bearings **(351)** are each formed from a U-shaped carrier **(353)** which is connected in the third axis of rotation **(D3)** in each case to a fork **(352)** which is suspended in the second axis of rotation (**D2**).

31. Sluice arrangement according to at least one of claims 18 to 30, **characterized in that** in each passage **(47, 48)** there is a seal **(470, 480)** which, together with the cover **(98)** of a temporarily pressed-on container **(9; 91, 92, 93)** and/or with a temporarily pressed-on lid **(51),** creates seals at the passages **(47, 48)** in the transition from the input station **(3)** to the process chamber (**43**).

32. Sluice arrangement according to at least one of claims 18 to 31, **characterized in that** a pivotable cantilever **(50)** of an apparatus **(5)** positioned in the process chamber **(43)** carries the lid (**51**).

33. Sluice arrangement according to at least one of claims 21 to 32, **characterized in that** the at least one manipulator **(6)** or also the additional manipulator **(6)** is designed as a robot.

34. Sluice arrangement according to at least one of claims 18 to 33, **characterized in that** the receptacles **(94)** of the containers **(9; 91, 92, 93)** have the shape of trough-shaped tubs and the articles **(960),** for example vials or syringes, are inserted in systematically arranged receiving contours of nests (**96**).

## Revendications

1. Procédé pour introduire des articles (960) dans des conditions aseptiques à partir d'emballages (9 ; 91, 92, 93) amenés en série dans une chambre de traitement (43) entourée d'un boîtier (49) d'une enceinte de confinement (4) au moyen d'un agencement de sas (1) positionné dans un espace d'installation (A), dans lequel :
a) un poste d'entrée (3) est placé en amont de la chambre de traitement (43) ;
b) un emballage individuel (9 ; 91, 92, 93) comprend :
ba) un récipient (94) avec un espace intérieur aseptique (940) et un passage (95) qui est fermé par un recouvrement (98) ; et
bb) les articles (960) stockés dans l'espace intérieur (940), qui doivent être traités dans la chambre de traitement (43) après ouverture du recouvrement (98),
**caractérisé par** les étapes de procédé successives dans le mode de production de l'agencement de sas (1) :
c) pour le transfert des articles (960) à partir des emballages (9 ; 91, 92, 93) dans la chambre de traitement (43), il est prévu au moins deux passages (47, 48) menant du poste d'entrée (3) dans la chambre de traitement (43), et chacun des passages (47, 48) est fermé d'une part par un couvercle (51) amené dans la chambre de traitement (43) et/ou d'autre part par un emballage (9 ; 91, 92, 93) amené dans le poste d'entrée (3) avec son recouvrement (98) tourné vers le passage (47, 48) ;
d) lorsque l'emballage (9 ; 91, 92, 93) est amené au passage (47, 48), la surface du recouvrement (98) tournée vers le passage (47, 48) est décontaminée avec l'environnement renfermé (8) au moyen d'un dispositif de décontamination (7) ;
e) après que la surface du recouvrement (98) tournée vers le passage (47, 48), y compris son environnement (8), a été décontaminée, le recouvrement (98) est ouvert pour transférer les articles (960) à partir du récipient (94) à travers son passage (95) dans la chambre de traitement (43) ; et
f) après avoir vidé le récipient (94), le couvercle (51) est d'abord ramené au passage (47, 48) concerné, pour retirer ensuite du passage (47, 48) le récipient (94) vidé de ses articles (960) ;
g) le couvercle (51) est utilisé en alternance sur les passages (47, 48).

2. Procédé selon la revendication 1, **caractérisé en ce que**
a) le dispositif de décontamination (7) est intégré dans le couvercle (51) ou disposé à l'extérieur du couvercle (51) ; et
b) l'élément d'émission du dispositif de décontamination (7) est disposé directement dans le couvercle (51) ou à l'extérieur du couvercle (51) dans un logement (70).

3. Procédé selon au moins l'une des revendications 1 et 2, **caractérisé en ce que** l'environnement renfermé (8) comprend le volume de gaz présent dans celui-ci et les surfaces baignées par le volume de gaz, à savoir les surfaces :
a) du couvercle (51) ;
b) du logement optionnel (70) ;
c) d'un joint optionnel (470, 480) ; et
d) des surfaces de paroi optionnellement exposées.

4. Procédé selon au moins l'une des revendications 1 à 3, **caractérisé en ce que**
a) la chambre de traitement (43) est équipée d'un manipulateur (6) ou en plus d'un deuxième manipulateur (6), sur les bras pivotants (60) desquels se trouve respectivement une tête d'outil (61) équipée d'outils, dans lequel :
b) les outils sont conçus pour ouvrir le recouvrement (98) et transférer ensuite les articles (960) à partir du récipient (94) à travers le passage (95) exposé dans la chambre de traitement (43) ; et
c) le premier manipulateur (6) avec ses outils est utilisé alternativement aux passages (47, 48) pour ouvrir le recouvrement (98) et transférer les articles (960) dans la chambre de traitement (43) ; ou
d) le premier manipulateur (6) et le deuxième manipulateur (6) sont utilisés chacun seulement à l'un des passages (47, 48) ou les deux manipulateurs (6) sont utilisés alternativement aux passages (47, 48).

5. Procédé selon au moins l'une des revendications 1 à 4, **caractérisé en ce que**
a) les outils comprennent un outil de coupe comme composant, destiné à ouvrir le recouvrement (98) ;
b) de préférence, au moins des parties de surface du recouvrement (98) et de l'enveloppe (99) sont constituées d'un tissu non tissé semi-perméable, tel que Tyvek^{®}, et le recouvrement (98), avant d'être retiré, conserve l'espace intérieur (940) du récipient (94) dans un état stérile et est scellé sur un bord (941) périphérique du passage (95) du récipient (94).

6. Procédé selon au moins l'une des revendications 1 à 5, **caractérisé en ce que**
a) un nid amovible (96) est stocké dans le récipient (94) pour recevoir une pluralité d'articles (960), lequel est transféré dans la chambre de traitement (43) en même temps que les articles (960) ; et
b) après avoir vidé le nid (96) des articles (960) dans la chambre de traitement (43), ce nid vidé (96) est évacué de la chambre de traitement (43) dans un récipient (94) suivant ou le même récipient (94) vidé pressé contre le passage (47, 48) concerné ; ou
c) les articles (960) sont transférés directement du nid (96) restant dans le récipient (94) dans la chambre de traitement (43).

7. Procédé selon au moins l'une des revendications 1 à 6, **caractérisé en ce que** le dispositif de décontamination est de préférence réalisé sous la forme d'une source de rayonnement, par exemple UVC, UV, E-Beam ou lumière pulsée, ou en variante d'un dispositif de fumigation, par exemple pour la nébulisation d'une solution de H₂O₂.

8. Procédé selon au moins l'une des revendications 1 à 7, **caractérisé en ce que** le poste d'entrée (3) est entouré d'un boîtier (39) qui présente un accès (37) menant à un poste d'approvisionnement (2) adjacent, dans lequel :
a) le poste d'entrée (3) fonctionne avec un flux de déplacement (LF) purifié et redressé, qui sort partiellement par l'accès (37) ;
b) au poste d'approvisionnement (2) sont livrés les emballages (9 ; 91, 92, 93) qui, à l'état de livraison, sont chacun entourés d'une enveloppe (99) afin de conserver de manière aseptique le volume intérieur (90) de l'emballage (9 ; 91, 92, 93) avec son contenu ; et
c) au poste d'approvisionnement (2), une ouverture pratiquée sur l'enveloppe (99), que l'on maintient fermée jusqu'à présent, est à présent libérée du côté tourné vers le flux de déplacement (LF) sortant de l'accès (37) afin d'introduire l'emballage individuel (9 ; 91, 92, 93) dans le poste d'entrée (3) ; ou
d) au poste d'approvisionnement (2), l'enveloppe (99) est séparée du côté tourné vers le flux de déplacement (LF) sortant de l'accès (37) afin d'introduire l'emballage individuel (9 ; 91, 92, 93) dans le poste d'entrée (3).

9. Procédé selon la revendication 8, **caractérisé en ce que**
a) le positionnement de l'emballage (9 ; 91, 92, 93) se trouvant dans l'enveloppe (99) d'abord encore fermée s'effectue sur un support (20) ;
b) un moyen d'avancement (22) est utilisé pour expulser l'emballage (9 ; 91, 92, 93) de l'enveloppe ouverte (99) tandis que le flux de déplacement (LF) s'écoule sur l'emballage (9 ; 91, 92, 93) ; et
c) l'enveloppe (99) est maintenue par un préhenseur (21), de préférence réalisé sous la forme d'une ventouse.

10. Procédé selon au moins l'une des revendications 1 à 9, **caractérisé en ce que**
a) les emballages (9 ; 91, 92, 93) introduits dans le poste d'entrée (3) sont amenés aux passages (47, 48) au moyen d'au moins un dispositif de transport (34, 35) ; et
b) ledit au moins un dispositif de transport (34, 35) repose de préférence sur le principe de la technique de lévitation magnétique.

11. Procédé selon la revendication 10, **caractérisé en ce que**
a) le transport des emballages (9 ; 91, 92, 93) à l'intérieur du poste d'entrée (3) s'effectue au moyen d'un premier dispositif de transport (34) ; tandis que
b) un deuxième dispositif de transport (35) provoque le pivotement des emballages (9 ; 91, 92, 93) avec le recouvrement (98) pressé contre le passage respectif (47, 48) ; et
c) le deuxième dispositif de transport (35) peut également être utilisé pour retirer les emballages traités (9 ; 91, 92, 93) du passage respectif (47, 48).

12. Procédé selon la revendication 11, **caractérisé en ce que** pour le positionnement des emballages (9 ; 91, 92, 93) au niveau du passage respectif (47, 48), le deuxième dispositif de transport (35) comprend un palier à cardan (351).

13. Procédé selon au moins l'une des revendications 11 et 12, **caractérisé en ce que** le deuxième dispositif de transport (35) est constitué de :
a) un axe (350) à travers lequel s'étend un premier axe de rotation (D1) ;
b) deux fourches (352) fixées l'une à côté de l'autre sur l'axe (350), qui peuvent chacune pivoter autour d'un deuxième axe de rotation (D2) qui s'étend perpendiculairement au premier axe de rotation (D1) ; et
c) un support (353) respectivement suspendu dans la fourche (352) correspondante et pouvant pivoter autour d'un troisième axe de rotation (D3), le premier axe de rotation (D1) et le troisième axe de rotation (D3) s'étendant parallèlement l'un à l'autre et les supports (353) étant configurés pour saisir par le dessous les bords (941) des récipients (94) lors du pivotement d'approche ou d'éloignement des passages (47, 48) ;
d) deux paliers à cardan (351) situés côte à côte sont formés chacun par un support en forme de U (353) qui est relié selon le troisième axe de rotation (D3) respectivement à une fourche (352) qui est suspendue selon le deuxième axe de rotation (D2).

14. Procédé selon au moins l'une des revendications 1 à 13, **caractérisé en ce que** dans chaque passage (47, 48) se trouve un joint d'étanchéité (470, 480) qui, avec le recouvrement (98) d'un emballage (9 ; 91, 92, 93) pressé temporairement et/ou avec un couvercle (51) pressé temporairement sur les passages (47, 48), assure l'étanchéité dans la transition entre le poste d'entrée (3) et la chambre de traitement (43).

15. Procédé selon au moins l'une des revendications 1 à 14, **caractérisé en ce que** le couvercle (51) est porté par un bras pivotant (50) d'un appareillage (5) positionné dans la chambre de traitement (43).

16. Procédé selon au moins l'une des revendications 4 à 15, **caractérisé en ce que** ledit au moins un manipulateur (6) ou également le manipulateur supplémentaire (6) sont réalisés sous la forme de robots.

17. Procédé selon au moins l'une des revendications 1 à 16, **caractérisé en ce que** les récipients (94) des emballages (9 ; 91, 92, 93) ont l'apparence de bacs en forme de cuve et les articles (960) sont par exemple des fioles, des flacons ou des seringues qui sont insérés dans des contours de réception disposés systématiquement de nids (96).

18. Agencement de sas pour introduire des articles (960) dans des conditions aseptiques à partir d'emballages (9 ; 91, 92, 93) amenés en série dans une chambre de traitement (43) entourée d'un boîtier (49) d'une enceinte de confinement (4) au moyen d'un agencement de sas (1) positionné dans un espace d'installation (A), dans lequel :
a) un poste d'entrée (3) est placé en amont de la chambre de traitement (43) ;
b) un emballage individuel (9 ; 91, 92, 93) comprend :
ba) un récipient (94) avec un espace intérieur aseptique (940) et un passage (95) qui est fermé par un recouvrement (98) ; et
bb) les articles (960) stockés dans l'espace intérieur (940), qui doivent être traités dans la chambre de traitement (43) après ouverture du recouvrement (98),
**caractérisé en ce que** :
c) pour le transfert des articles (960) des emballages (9 ; 91, 92, 93) dans la chambre de traitement (43), il est prévu au moins deux passages (47, 48) menant du poste d'entrée (3) dans la chambre de traitement (43), et chacun des passages (47, 48) est fermé d'une part par un couvercle (51) amené dans la chambre de traitement (43) et/ou d'autre part par un emballage (9 ; 91, 92, 93) amené dans le poste d'entrée (3) avec son recouvrement (98) tourné vers le passage (47, 48) ;
d) lorsque l'emballage (9 ; 91, 92, 93) est amené au passage (47, 48), la surface du recouvrement (98) tournée vers le passage (47, 48) est décontaminée avec l'environnement renfermé (8) au moyen d'un dispositif de décontamination (7) ;
e) après que la surface du recouvrement (98) tournée vers le passage (47, 48), y compris son environnement (8), a été décontaminée, le recouvrement (98) peut être ouvert et les articles (960) peuvent être transférés du récipient (94) à travers son passage (95) dans la chambre de traitement (43) ; et
f) le couvercle (51) peut à nouveau être appliqué sur le passage (47, 48) concerné, avec lequel le récipient (94) vidé est en contact, et le récipient (94) vide peut ainsi être retiré du passage (47, 48) ;
g) le couvercle (51) étant utilisé en alternance sur les passages (47, 48).

19. Agencement de sas selon la revendication 18, **caractérisé en ce que**
a) le dispositif de décontamination (7) est intégré dans le couvercle (51) ou disposé à l'extérieur du couvercle (51) ; et
b) l'élément d'émission du dispositif de décontamination (7) est disposé directement dans le couvercle (51) ou à l'extérieur du couvercle (51) dans un logement (70).

20. Agencement de sas selon au moins l'une des revendications 18 et 19, **caractérisé en ce que** l'environnement renfermé (8) comprend le volume de gaz présent dans celui-ci et les surfaces baignées par le volume de gaz, à savoir les surfaces :
a) du couvercle (51) ;
b) du logement optionnel (70) ;
c) d'un joint optionnel (470, 480) ; et
d) des surfaces de paroi optionnellement exposées.

21. Agencement de sas selon au moins l'une des revendications 18 à 20, **caractérisé en ce que**
a) la chambre de traitement (43) est équipée d'un manipulateur (6) ou en plus d'un deuxième manipulateur (6), sur les bras pivotants (60) desquels se trouve respectivement une tête d'outil (61) équipée d'outils, dans lequel :
b) les outils sont conçus pour ouvrir le recouvrement (98) et transférer ensuite les articles (960) à partir du récipient (94) à travers le passage (95) exposé dans la chambre de traitement (43) ; et
c) le premier manipulateur (6) avec ses outils est utilisé alternativement aux passages (47, 48) pour ouvrir le recouvrement (98) et transférer les articles (960) dans la chambre de traitement (43) ; ou
d) le premier manipulateur (6) et le deuxième manipulateur (6) sont utilisés chacun seulement à l'un des passages (47, 48) ou les deux manipulateurs (6) sont utilisés alternativement aux passages (47, 48).

22. Agencement de sas selon au moins l'une des revendications 18 à 21, **caractérisé en ce que**
a) les outils comprennent un outil de coupe comme composant, destiné à ouvrir le recouvrement (98) ;
b) de préférence, au moins des parties de surface du recouvrement (98) et de l'enveloppe (99) sont constituées d'un tissu non tissé semi-perméable, tel que Tyvek^{®}, et le recouvrement (98), avant d'être retiré, conserve l'espace intérieur (940) du récipient (94) dans un état stérile et est scellé sur un bord (941) périphérique du passage (95) du récipient (94).

23. Agencement de sas selon au moins l'une des revendications 18 à 22, **caractérisé en ce que**
a) un nid amovible (96) est stocké dans le récipient (94) pour recevoir une pluralité d'articles (960), lequel peut être transféré dans la chambre de traitement (43) en même temps que les articles (960) ; et
b) le nid (96) vidé des articles (960) peut être évacué de la chambre de traitement (43) dans un récipient (94) suivant ou le même récipient (94) vidé arrimé au passage (47, 48) concerné ; ou
c) les articles (960) peuvent être transférés directement du nid (96) restant dans le récipient (94) dans la chambre de traitement (43).

24. Agencement de sas selon au moins l'une des revendications 18 à 23, **caractérisé en ce que** le dispositif de décontamination est de préférence réalisé sous la forme d'une source de rayonnement, par exemple UVC, UV, E-Beam ou lumière pulsée, ou en variante d'un dispositif de fumigation, par exemple pour la nébulisation d'une solution de H₂O₂.

25. Agencement de sas selon au moins l'une des revendications 18 à 24, **caractérisé en ce que** le poste d'entrée (3) est entouré d'un boîtier (39) qui présente un accès (37) menant à un poste d'approvisionnement (2) adjacent, dans lequel :
a) le poste d'entrée (3) peut fonctionner avec un flux de déplacement (LF) purifié et redressé, qui sort partiellement par l'accès (37) ;
b) le poste d'approvisionnement (2) est destiné à la livraison des emballages (9 ; 91, 92, 93) qui, à l'état de livraison, sont chacun entourés d'une enveloppe (99) afin de conserver de manière aseptique le volume intérieur (90) de l'emballage (9 ; 91, 92, 93) avec son contenu ; et
c) au poste d'approvisionnement (2), une ouverture pratiquée sur l'enveloppe (99), qui reste jusqu'alors fermée au moyen d'un outil appliqué, est à présent libérée du côté tourné vers le flux de déplacement (LF) sortant de l'accès (37) par desserrage de l'outil, de sorte que l'emballage individuel (9 ; 91, 92, 93) peut être introduit dans le poste d'entrée (3) ; ou
d) au poste d'approvisionnement (2), l'enveloppe (99) peut être séparée du côté tourné vers le flux de déplacement (LF) sortant de l'accès (37), de sorte que l'emballage individuel (9 ; 91, 92, 93) peut être introduit dans le poste d'entrée (3).

26. Agencement de sas selon la revendication 25, **caractérisé en ce que**
a) un support (20) est prévu pour le positionnement de l'emballage (9 ; 91, 92, 93) se trouvant dans l'enveloppe (99) d'abord encore fermée ;
b) un moyen d'avancement (22) sert à expulser l'emballage (9 ; 91, 92, 93) de l'enveloppe ouverte (99) tandis que le flux de déplacement (LF) s'écoule sur l'emballage (9 ; 91, 92, 93) ; et
c) un dispositif de préhension (21), de préférence réalisé sous la forme d'une ventouse, sert à retenir l'enveloppe (99).

27. Agencement de sas selon au moins l'une des revendications 18 à 26, **caractérisé en ce que**
a) au moins un dispositif de transport (34, 35) est prévu pour amener les emballages (9 ; 91, 92, 93) introduits dans le poste d'entrée (3) aux passages (47, 48) ; et
b) ledit au moins un dispositif de transport (34, 35) repose sur le principe de la technique de lévitation magnétique.

28. Agencement de sas selon la revendication 27, **caractérisé en ce que**
a) un premier dispositif de transport (34) sert à transporter les emballages (9 ; 91, 92, 93) à l'intérieur du poste d'entrée (3) ; tandis que
b) un deuxième dispositif de transport (35) est destiné à faire pivoter les emballages (9 ; 91, 92, 93) avec le recouvrement (98) pressé contre le passage respectif (47, 48) ; et
c) le deuxième dispositif de transport (35) peut également être utilisé pour retirer les emballages traités (9 ; 91, 92, 93) du passage respectif (47, 48).

29. Agencement de sas selon la revendication 28, **caractérisé en ce que** pour le positionnement des emballages (9 ; 91, 92, 93) au niveau du passage respectif (47, 48), le deuxième dispositif de transport (35) comprend un palier à cardan (351).

30. Agencement de sas selon au moins l'une des revendications 28 et 29, **caractérisé en ce que** le deuxième dispositif de transport (35) est constitué de :
a) un axe (350) à travers lequel s'étend un premier axe de rotation (D1) ;
b) deux fourches (352) fixées l'une à côté de l'autre sur l'axe (350), qui peuvent chacune pivoter autour d'un deuxième axe de rotation (D2) qui s'étend perpendiculairement au premier axe de rotation (D1) ; et
c) un support (353) respectivement suspendu dans la fourche (352) correspondante et pouvant pivoter autour d'un troisième axe de rotation (D3), le premier axe de rotation (D1) et le troisième axe de rotation (D3) s'étendant parallèlement l'un à l'autre et les supports (353) étant configurés pour saisir par le dessous les bords (941) des récipients (94) lors du pivotement d'approche ou d'éloignement des passages (47, 48) ;
d) deux paliers à cardan (351) situés côte à côte sont formés chacun par un support en forme de U (353) qui est relié selon le troisième axe de rotation (D3) respectivement à une fourche (352) qui est suspendue selon le deuxième axe de rotation (D2).

31. Agencement de sas selon au moins l'une des revendications 18 à 15, **caractérisé en ce que** dans chaque passage (47, 48) se trouve un joint d'étanchéité (470, 480) qui, avec le recouvrement (98) d'un emballage (9 ; 91, 92, 93) pressé temporairement et/ou avec un couvercle (51) pressé temporairement sur les passages (47, 48), assure l'étanchéité dans la transition entre le poste d'entrée (3) et la chambre de traitement (43).

32. Agencement de sas selon au moins l'une des revendications 18 à 31, **caractérisé en ce qu'**un bras pivotant (50) d'un appareillage (5) positionné dans la chambre de traitement (43) porte le couvercle (51).

33. Agencement de sas selon au moins l'une des revendications 21 à 32, **caractérisé en ce que** ledit au moins un manipulateur (6) ou également le manipulateur supplémentaire (6) sont réalisés sous la forme de robots.

34. Agencement de sas selon au moins l'une des revendications 18 à 33, **caractérisé en ce que** les récipients (94) des emballages (9 ; 91, 92, 93) ont l'apparence de bacs en forme de cuve et les articles (960) sont par exemple des fioles, des flacons ou des seringues qui sont insérés dans des contours de réception disposés systématiquement de nids (96).
